# EUROPEAN PATENT SPECIFICATION

(11) **EP 4 423 056 B1**
(45) Date of publication and mention of the grant of the patent: **06.05.2026**
(21) Application number: 22818156.6
(22) Date of filing: 26.10.2022
(51) Int. Cl.: C07C 229/16, C07B 59/00

(54) **SYNTHESIS OF RESTRAINED COMPLEXING AGENTS**
SYNTHESE VON EINGESCHRÄNKTEN KOMPLEXBILDNERN
SYNTHÈSE D'AGENTS COMPLEXANTS RESTREINTS

(30) Priority: 27.10.2021 US 202163272547 P
(43) Date of publication of application: 04.09.2024
(73) Proprietor: GENENTECH, INC., South San Francisco, CA 94080 (US)
(72) Inventor: WONG, Nicholas, South San Francisco, California 94080 (US); GOSSELIN, Francis, South San Francisco, California 94080 (US)
(74) Representative: Mewburn Ellis LLP
(86) International application number: PCT/US2022/078702
(87) International publication number: WO 2023/076938

(56) References cited:
- WO-A2-2016/065435

## Description

### CROSS REFERENCE TO RELATED APPLICATIONS

This application claims priority to and the benefit of U.S. Application No. 63/272,547 filed on October 27, 2021.

### FIELD

The present application is related to methods of preparing restrained complexing agents (RESCA) and RESCA compounds and salts thereof.

### BACKGROUND

Proteins that bind molecules that are important in diagnosis, disease monitoring, and visualization ("marker-binding proteins") can have associated or attached a detectable label (e.g., radioactive label or fluorescent label) that permits visualizing the molecule and thus its location and quantity (qualitative or quantitative). In some cases, a detectable label is directly associated or attached to the marker-binding protein, but in other cases, detectable labels are attached to marker proteins via linking molecules ("linkers"), which may be uncleavable or cleavable (thus allowing for controlled association of the marker-binding protein with its detectable label). Linkers may associate with the detectable label in a variety of ways, including direct conjugation to chelation, the latter suited for some metal-containing detectable labels. However, linkers can require conditions for conjugation to the marker protein that are incompatible with a particular marker protein, or are inefficient, or require multiple reagents.

Restrained complexing agent (RESCA) is a class of chelator molecules that have been successfully used to label biomolecules with ¹⁸F (fluorine-18) and facilitate PET (positron emission tomography) imaging that could be useful as a diagnostic and/or therapeutic tool for a variety of diseases and conditions. The properties of ¹⁸F, particularly its modest half-life, make it an attractive option for imaging technologies compared to other radionuclides, such as ⁸⁹Zr (zirconium-89) and ¹²⁴I (iodine-124). The use of aluminum monofluoride ({Al¹⁸F}²⁺) as the source of ¹⁸F has emerged as a strategy that allows ¹⁸F labeling via RESCA in aqueous media and at lower temperatures compared to previously known methods of ¹⁸F labeling, for example as described in WO 2016/065435. Despite the potential applications for RESCA, the laborious, low-yield synthesis is a potential barrier to widespread development and use of the RESCA-Al¹⁸F labeling strategy. An improved process for synthesizing RESCA would be useful for the continued development of the RESCA-Al¹⁸F labeling method and its various applications.

### BRIEF SUMMARY

The present application provides methods of preparing RESCA compounds, RESCA compounds and salts thereof as defined in the appended claims.

Provided herein is a method of preparing a compound of Formula (I): 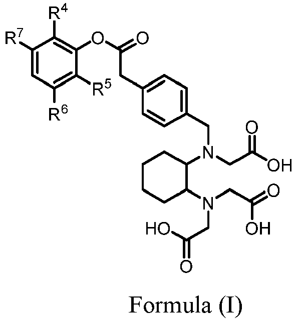
a diastereomer thereof, a salt thereof, a solvate thereof, or a combination of any of the foregoing,
wherein the method comprises:
   (a) a step (1-1) comprising reacting a compound of Formula (Ia): 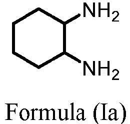
      or a diastereomer thereof with a compound of Formula (Ib): 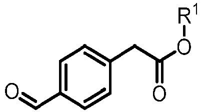 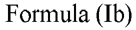
      in a solvent and in the presence of a reducing agent to form a compound of Formula (Ic): 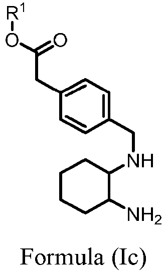
      or a diastereomer thereof;
   (b) a step (1-2) comprising reacting the compound of Formula (Ic), or a salt thereof, with a compound of Formula (Id) 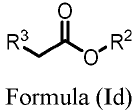
      in the presence of a base and in a solvent,
      to form a compound of Formula (Ie): 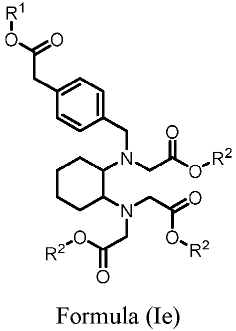
      or a diastereomer thereof;
   (c) a step (1-3) comprising reacting the compound of Formula (Ie) with a base in a solvent to form a compound of Formula (If): 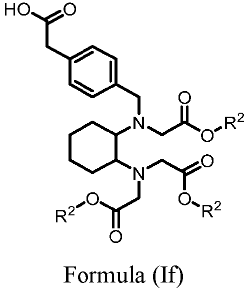 or a diastereomer thereof;
   (d) a step (1-4) comprising reacting the compound of Formula (If) with a compound of Formula (1g): 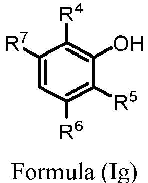
      in a solvent and in the presence of a carboxyl activating reagent, to form a compound of Formula (Ih): 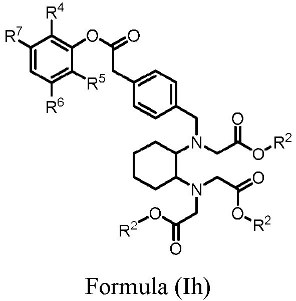
      or a diastereomer thereof; and
   (e) a step (1-5) comprising reacting the compound of Formula (Ih) with an acid to form a compound of Formula (I), a diastereomer thereof, a salt thereof, or a solvate thereof, or a combination of any of the foregoing,
      wherein
      R¹ and R² are each independently C₁-C₆ alkyl;
      R³ is halo; and
      R⁴, R⁵, R⁶ and R⁷ are each independently halo or hydrogen.

In some embodiments, the solvent in the step (1-1) comprises an alcohol. In some embodiments, the solvent is ethanol. In some embodiments, the reducing agent in the step (1-1) is a hydride. In some embodiments, the reducing agent is NaBH(OAc)₃.

In some embodiments, step (1-1) further comprises purifying the compound of Formula (Ic) by column chromatography.

In some embodiments, the solvent in the step (1-2) is a polar organic solvent. In some embodiments, the solvent is CH₃CN. In some embodiments, the base in the step (1-2) is an organic base. In some embodiments, the organic base is an amine base. In some embodiments, the amine base is N,N-diisopropylethylamine (DIPEA).

In some embodiments, the step (1-2) further comprises purifying the compound of Formula (1e) by column chromatography.

In some embodiments, the solvent in the step (1-3) is an aqueous solvent. In some embodiments, the solvent further comprises tetrahydrofuran (THF). In some embodiments, the base in the step (1-3) is KOH.

In some embodiments, the solvent in the step (1-4) is a halogenated hydrocarbon. In some embodiments, the solvent is CH₂Cl₂. In some embodiments, the carboxyl activating reagent is 1-ethyl-3-(3-dimethylaminopropyl)carbodiimide (EDCI).

In some embodiments, the acid in the step (1-5) is trifluoroacetic acid.

In some embodiments, the step (1-5) further comprises purifying the compound of Formula (Ih) by preparative High Performance Liquid Chromatography.

In some embodiments of any of the preceding formulae, R¹ is methyl. In some embodiments, R² is tert-butyl. In some embodiments, R³ is Br. In some embodiments, R⁴, R⁵, R⁶ and R⁷ are each F. In some embodiments, the compound of Formula (I) is Compound 1:

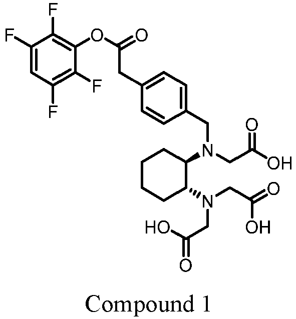

Provided herein is a method of preparing a compound of Formula (I): 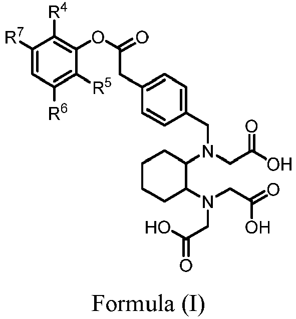
a diastereomer thereof, a salt thereof, or a solvate thereof, or a combination of any of the foregoing,
wherein the method comprises:
   (a) a step (2-1) comprising reacting a compound of Formula (Ia): 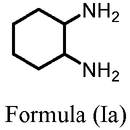
      with a compound of Formula (Ib): 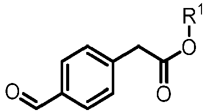 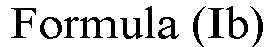
      in a solvent and in the presence of a reducing agent to form a compound of Formula (Ic): 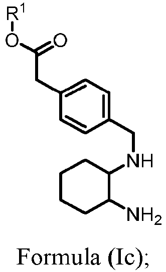
   (b) a step (2-2) comprising reacting a compound of Formula (Ic) with an acid to produce a salt of Formula (Ic);
   (c) a step (2-3) comprising reacting the salt of Formula (Ic) with a compound of Formula (Id): 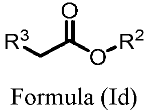
      in the presence of a base and in a solvent,
      to form a compound of Formula (Ie): 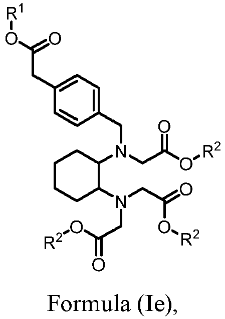
      a diastereomer thereof, or a salt thereof;
   (d) a step (2-4) comprising reacting a compound of Formula (Ie) with a base in a solvent to form a compound of Formula (2f): 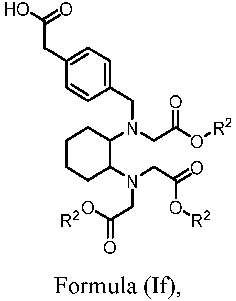 a diastereomer thereof, or a salt thereof; and
   (e) a step (2-5) comprising reacting the compound of Formula (If) or salt thereof with a compound of Formula (Ig): 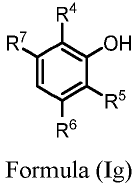
      in a solvent and in the presence of a carboxyl activating reagent, to form a compound of Formula (Ih): 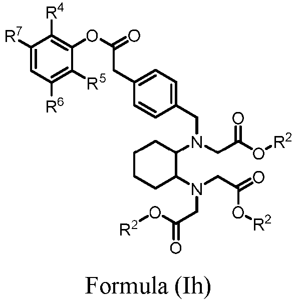
      a diastereomer thereof, or a salt thereof; and
   (f) a step (2-6) comprising reacting the compound of Formula (Ih) with an acid to form a compound of Formula (I), a diastereomer thereof, a salt thereof, a solvate thereof, or a combination of any of the foregoing,
      wherein
      R¹ and R² are each independently C₁-C₆ alkyl;
      R³ is halo; and
      R⁴, R⁵, R⁶, and R⁷ are each independently halo or hydrogen.

In some embodiments, the solvent in the step (2-1) comprises an alcohol. In some embodiments, the alcohol is ethanol. In some embodiments, the reducing agent in the step (2-1) comprises a hydride. In some embodiments, the reducing agent is NaBH(OAc)₃.

In some embodiments, the acid in the step (2-2) is orotic acid. In some embodiments, the step (2-2) provides a salt of Formula (Ic) having the following structure:

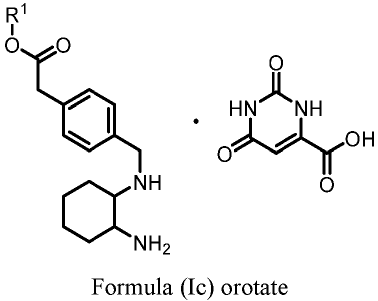

In some embodiments, the step (2-2) comprises crystallizing the salt of Formula (Ic), or a diastereomer thereof, as an orotate salt.

In some embodiments, the acid in step (2-2) is fumaric acid. In some embodiments, the step (2-2) provides a salt of Formula (Ic) having the following structure:

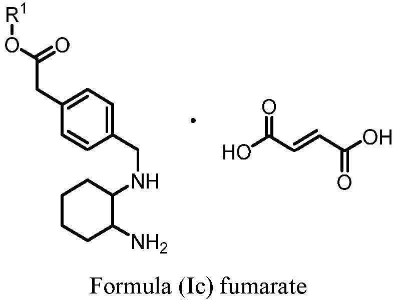

In some embodiments, the step (2-2) further comprises crystallizing the salt of Formula (Ic), or a diastereomer thereof, as fumarate salt.

In some embodiments, the base in the step (2-3) is K₂CO₃. In some embodiments, the solvent in the step (2-3) comprises toluene. In some embodiments, the solvent in the step (2-3) comprises water.

In some embodiments, the base in the step (2-4) is KOH. In some embodiments, the solvent in the step (2-4) comprises THF. In some embodiments, the solvent in the step (2-4) comprises water.

In some embodiments, the step (2-4) provides a salt of Formula (If) as an HCl salt, and wherein the step (2-4) further comprises crystallizing the salt of Formula (If).

In some embodiments, the solvent in the step (2-5) is a halogenated hydrocarbon. In some embodiments, the halogenated hydrocarbon is CH₂Cl₂. In some embodiments, the carboxyl activating reagent of the step (2-5) is EDCI.

In some embodiments, the solvent in the step (2-6) is dioxane. In some embodiments, the acid in the step (2-6) is HCl.

In some embodiments, the step (2-6) provides a salt of Formula (I), or a solvate thereof, having the following structure:

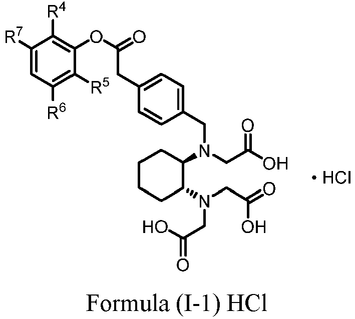

In some embodiments, the step (2-6) further comprises crystallizing the salt of Formula (I). In some embodiments in any of the preceding formulae, R¹ is methyl. In some embodiments, R² is tert-butyl. In some embodiments, R³ is Br. In some embodiments, R⁴, R⁵, R⁶, and R⁷ are F.

In some embodiments, the salt of the solvate of Formula (I) has the structure:

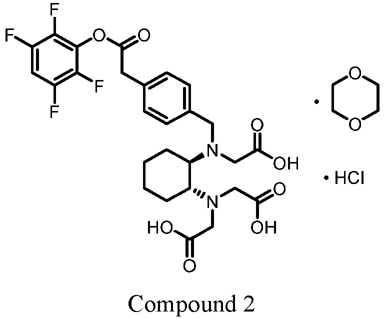

Described herein is a composition comprising one or more diastereomers of compound of Formula (I), salts thereof, or solvates thereof: 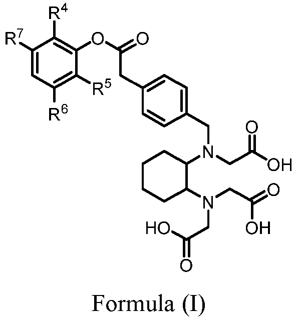 produced by a method as described herein.

The composition may comprise at least about 90 % (weight/weight) of a trans diastereomer of a compound of Formula (I). The trans diastereomer may have the structure:

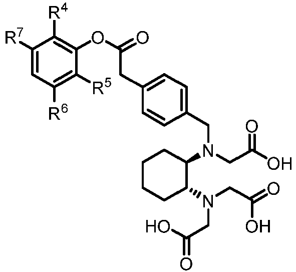

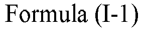

R⁴, R⁵, R⁶, and R⁷ may be F.

A salt of Formula (I), or a solvate thereof, is described herein, wherein the salt of Formula (I) has the structure: 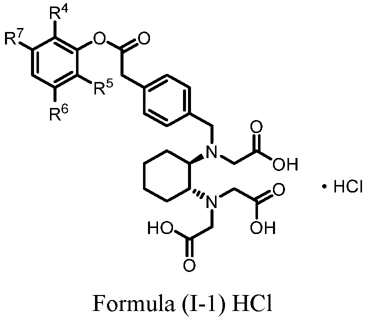 produced by a method as described herein. R⁴, R⁵, R⁶, and R⁷ may be F.

Provided herein is a compound of Formula (Ie): 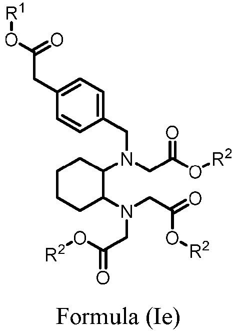
a diastereomer thereof, or a salt thereof,
wherein R¹ and R² are each independently C₁-C₆ alkyl.

Provided herein is a compound of Formula (Ic): 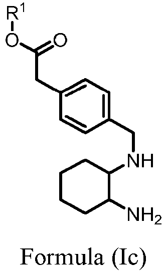
a diastereomer thereof, or a salt thereof,
wherein R¹ is C₁-C₆ alkyl.

Described herein is a composition comprising a complex comprising the compound prepared using a method described herein and a metal halide. The metal halide may be {Al¹⁸F}²⁺. At least 90% (weight/weight) of the compound in the composition may have the following structure: 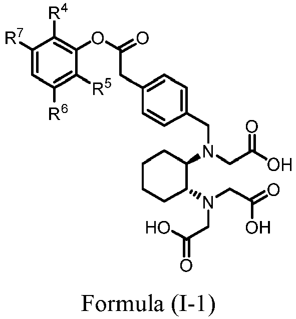 R⁴, R⁵, R⁶, and R⁷ may be F. The composition may further comprise a polypeptide, wherein the complex is conjugated to the polypeptide. The polypeptide may be an antibody or antibody fragment. The antibody fragment may be selected from the group consisting of a VHH, a scFv, a minibody, a Fab, a Fab', and a Fv fragment. The polypeptide may be a non-antibody scaffold protein. The non-antibody scaffold protein may be selected from the group consisting of an affibody, an affilin, a knottin, a fibronectin, an anticalin, an atrimer, an avimer, an FN3 scaffold, a fynomer, a Kunitz domain, a pronectin, an OBody, and a DARPin. The composition may be a pharmaceutical composition suitable for administration to a subject. The pharmaceutical composition may be suitable for intravenous administration. The subject may be a human.

Described herein is a method of preparing a labeled agent comprising a polypeptide and a ¹⁸F radionuclide, comprising:
(a) contacting the polypeptide with the compound prepared using a method described herein under a condition that allows conjugation of the complex to the polypeptide to provide a conjugate; and
(b) contacting the conjugate with an aluminum fluoride complex comprising ¹⁸F to provide the labeled agent.

Provided herein is a crystalline salt of a compound with the structure:

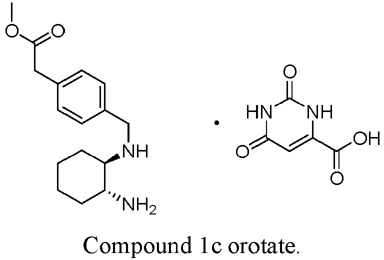

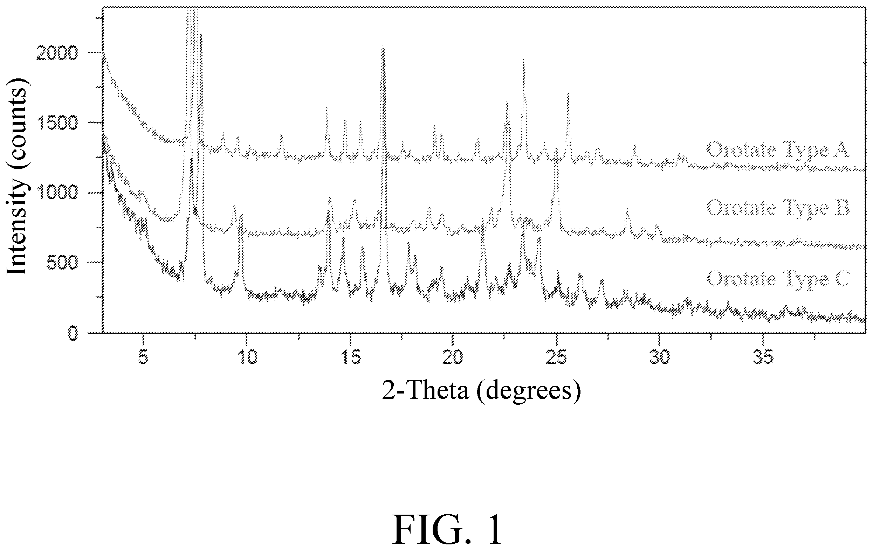

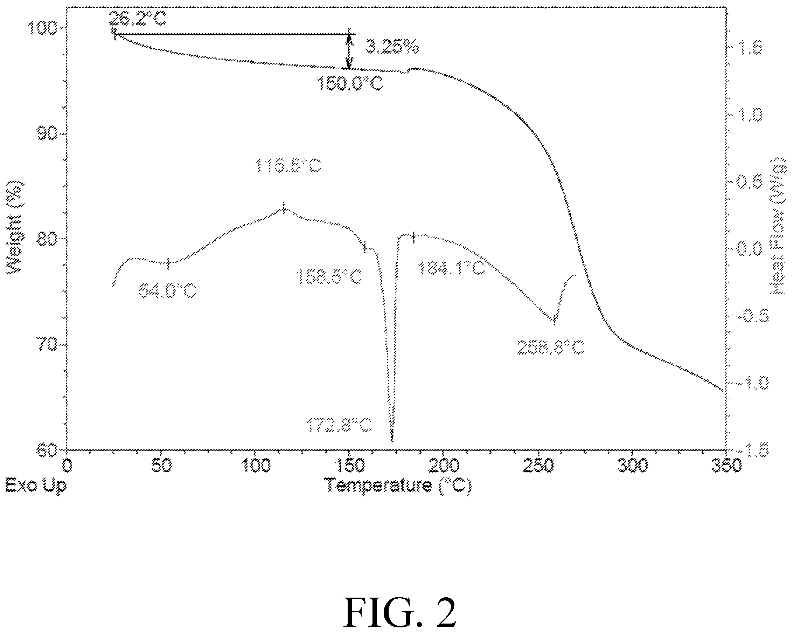

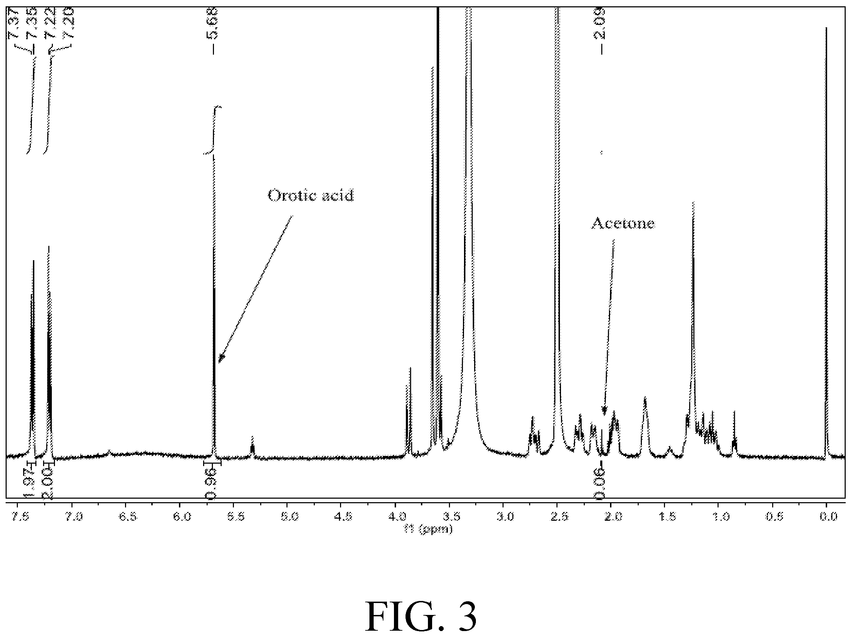

In some embodiments, the salt has at least one of:
(a) an X-ray powder diffraction pattern with characteristic peaks at about 7.5° 2-Theta, about 13.9° 2-Theta, about 16.6° 2-Theta, about 23.4° 2-Theta, and about 25.6° 2-Theta;
(b) an X-ray powder diffraction pattern substantially the same as the "Orotate Type A" pattern in FIG. 1;
(c) a thermo-gravimetric analysis thermogram showing a mass loss of about 3.3% between 20 °C and 150 °C;
(d) a thermo-gravimetric analysis thermogram substantially the same as shown in FIG. 2;
(e) a differential scanning calorimetry thermogram showing an endothermic peak at about 172.8 °C;
(f) a differential scanning calorimetry thermogram substantially the same as shown in FIG. 2;
(g) an ¹H nuclear magnetic resonance spectrum with a characteristic peak at about 5.68 ppm; or
(h) an ¹H nuclear magnetic resonance spectrum substantially the same as shown in FIG. 3.

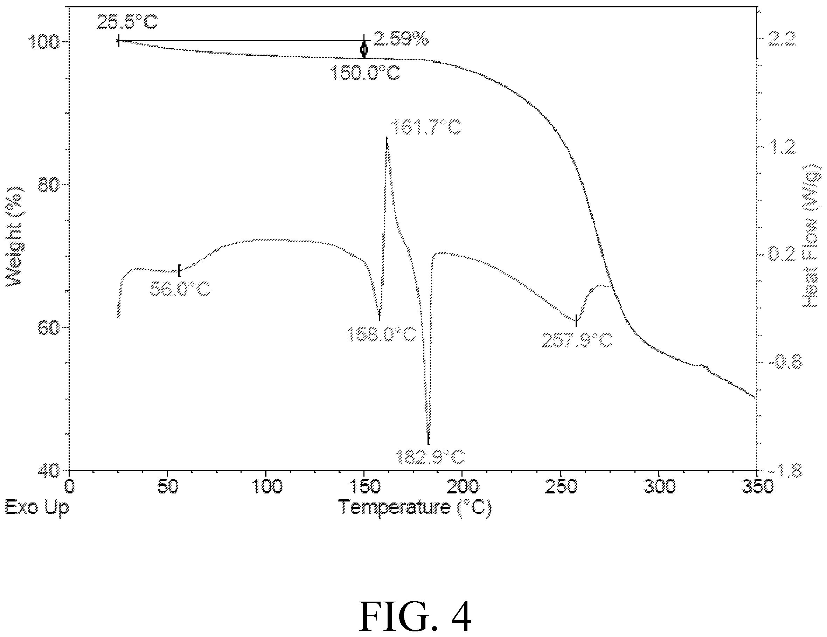

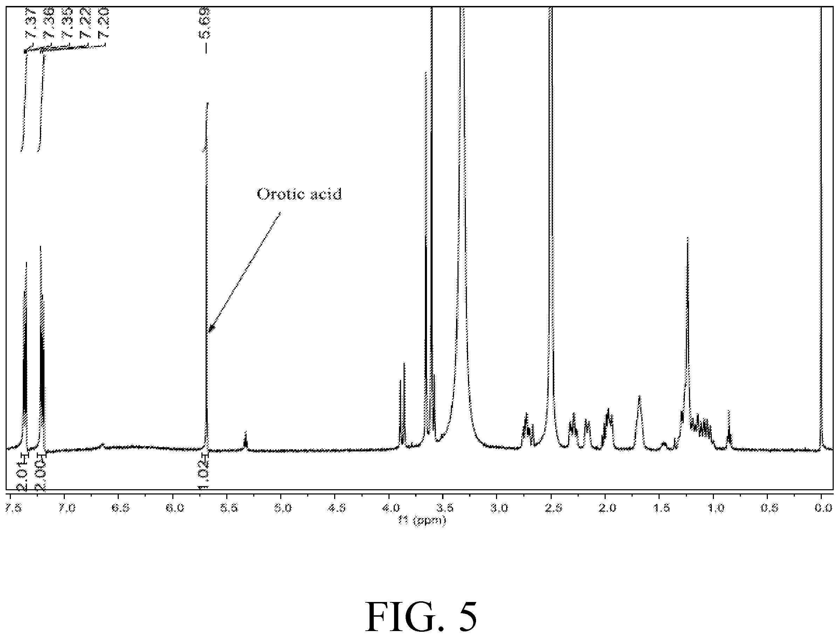

In some embodiments, provided herein is a crystalline salt of a compound with the structure: 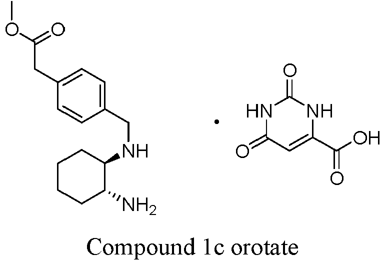 wherein the salt has at least one of:
(a) an X-Ray powder diffraction pattern with characteristic peaks at about 7.2° 2-Theta, about 14.0° 2-Theta, about 15.2° 2-Theta, about 22.7° 2-Theta, and about 25.0° 2-Theta;
(b) an X-Ray powder diffraction pattern substantially the same as the "Orotate Type C" pattern in FIG. 1;
(c) a thermo-gravimetric analysis thermogram showing a mass loss of about 2.6% between 20 °C and 150 °C;
(d) a thermo-gravimetric analysis thermogram substantially the same as shown in FIG. 4;
(e) a differential scanning calorimetry thermogram showing an exothermic peak at about 161.7 °C and an endothermic peak at about 182.9 °C;
(f) a differential scanning calorimetry thermogram substantially the same as shown in FIG. 4;
(g) an ¹H nuclear magnetic resonance spectrum with a characteristic peak at about 5.69 ppm; and
(h) an ¹H nuclear magnetic resonance spectrum substantially the same as shown in FIG. 5.

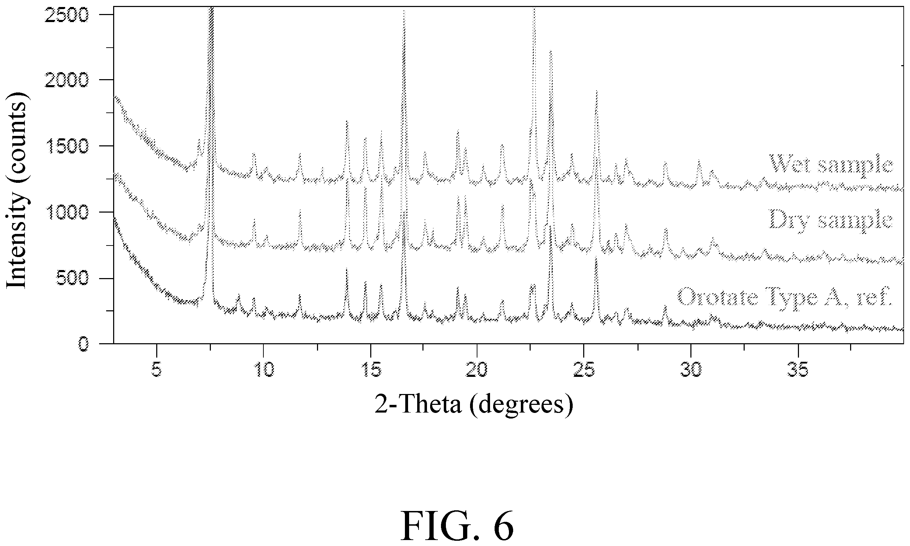

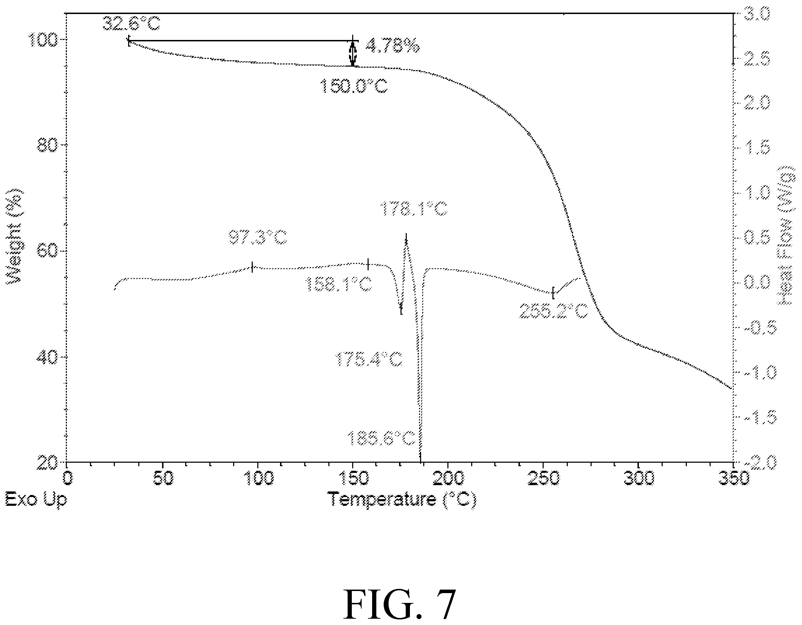

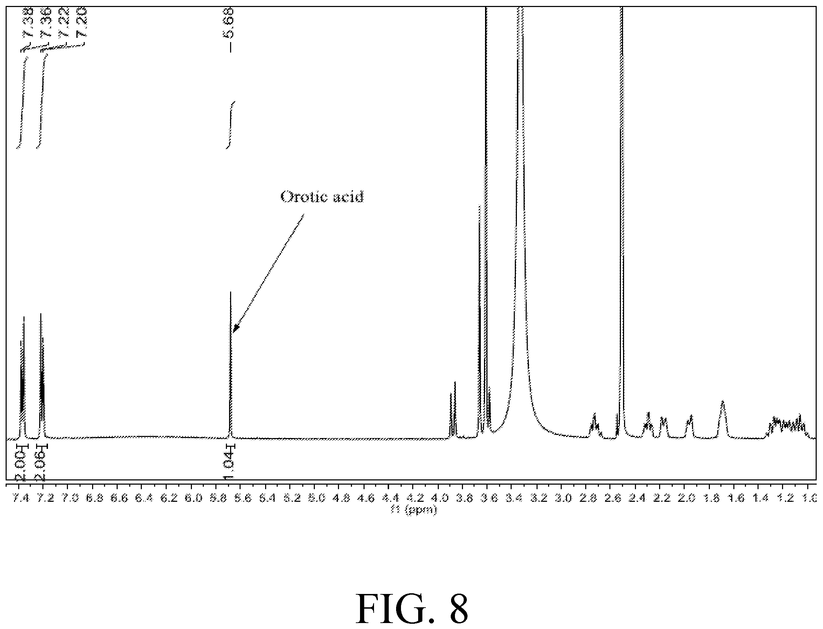

In some embodiments, provided herein is a crystalline salt of a compound with the structure: 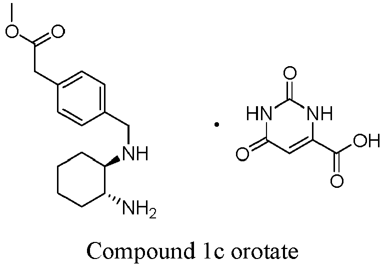 wherein the salt has at least one of:
(a) an X-Ray powder diffraction pattern with characteristic peaks at about 7.3° 2-Theta, about 7.8° 2-Theta, about 9.7° 2-Theta, about 13.9° 2-Theta, about 16.7° 2-Theta, about 21.4° 2-Theta, about 23.4° 2-Theta;
(b) an X-Ray powder diffraction pattern substantially the same as either "Wet Sample" or "Dry Sample" as shown in FIG.6;
(c) a thermo-gravimetric analysis thermogram showing a mass loss of about 4.8% between 20 °C and 150 °C;
(d) a thermo-gravimetric analysis thermogram substantially the same as shown in FIG. 7;
(e) a differential scanning calorimetry thermogram showing an exothermic peak at about 178.1 °C and an endothermic peak at about 185.6 °C;
(f) a differential scanning calorimetry thermogram substantially the same as shown in FIG. 7;
(g) an ¹H nuclear magnetic resonance spectrum with a characteristic peak at about 5.68 ppm; and
(h) an ¹H nuclear magnetic resonance spectrum substantially the same as shown in FIG. 8.

Provided herein is a crystallized salt of a compound with the structure: 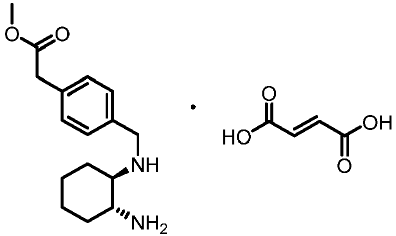 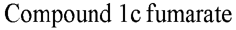 wherein the salt has at least one of:
(a) an X-Ray powder diffraction pattern with characteristic peaks at 7.9° 2-Theta, 15.8° 2-Theta, 18.6° 2-Theta, 19.3° 2-Theta, 20.1° 2-Theta, 21.0° 2-Theta, and 24.7° 2-Theta;
(b) a thermo-gravimetric analysis thermogram showing a mass loss of 2.4% between 20 °C and 140 °C;
(c) a differential scanning calorimetry thermogram showing an endothermic peak at 154.7 °C; and
(d) an ¹H nuclear magnetic resonance spectrum with a characteristic peak at 6.39 ppm.

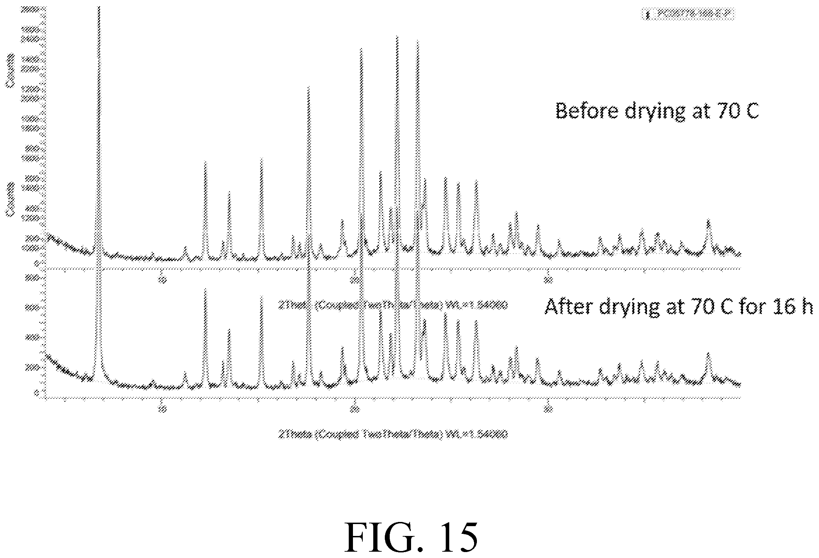

Described herein is a crystallized solvate of a salt of a compound with the structure: 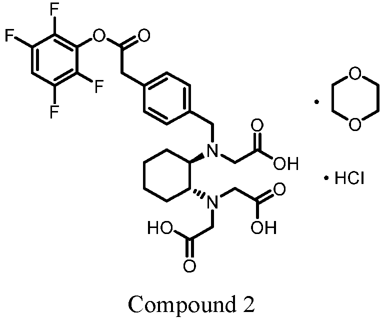 wherein the solvate of the salt has at least one of:
(a) an X-Ray powder diffraction pattern with characteristic peaks at about 6.8° 2-Theta, about 17.6° 2-Theta, about 20.3° 2-Theta, about 22.2° 2-Theta, and about 23.3° 2-Theta; and
(b) an X-Ray powder diffraction pattern substantially the same as shown in FIG. 15.

Described herein is a restrained complexing agent comprising
(i) a compound prepared using a method as provided herein; or
(ii) a crystallized salt or solvate as described herein.

Described herein is a label moiety comprising a detectable label comprising a metal halide conjugated or chelated to a linker comprising
(i) the compound prepared using a method as provided herein; or
(ii) a crystallized salt or solvate as described herein.

Described herein is a label conjugated marker-binder protein comprising a detectable label comprising a metal halide linked to said marker-binder protein by a linker comprising
(i) the compound prepared using a method as provided herein; or
(ii) a crystallized salt or solvate as described herein.

Described herein is a method of visualizing a molecule, said method comprising exposing said molecule to a label conjugated marker-binder protein comprising a detectable label comprising a metal halide linked to said marker-binder protein by a linker comprising
(i) the compound prepared using a method as provided herein; or
(ii) a crystallized salt or solvate as described herein,

wherein said marker-binder protein binds said molecule; and
detecting the label.

### BRIEF DESCRIPTION OF THE DRAWINGS

FIG. 1 is a collection of XRPD (X-ray powder diffraction) patterns for samples of a compound of Compound 1c orotate.
FIG. 2 depicts TGA (thermos-gravimetric analysis) and DSC (differential scanning calorimetry) thermograms for a compound of Compound 1c orotate (Orotate Type A).
FIG. 3 is an ¹H NMR (nuclear magnetic resonance) spectrum of Compound 1c orotate (Orotate Type A).
FIG. 4 depicts TGA and DSC thermograms for Compound 1c orotate (Orotate Type C).
FIG. 5 is an ¹H NMR spectrum of Compound 1c orotate (Orotate Type C).
FIG. 6 is a collection of XRPD patterns for samples Compound 1c orotate (re-prepared Orotate Type A).
FIG. 7 depicts TGA and DSC thermograms for Compound 1c orotate (re-prepared Orotate Type A).
FIG. 8 is an ¹H NMR spectrum of Compound 1c orotate (re-prepared Orotate Type A).
FIG. 9 is a collection of XRPD patterns for samples Compound 1c fumarate (Fumarate Type A).
FIG. 10 depicts TGA and DSC thermograms for Compound 1c fumarate (Fumarate Type A).
FIG. 11 is an ¹H NMR spectrum of Compound 1c fumarate (Fumarate Type A).
FIG. 12 is a collection of XRPD patterns for samples Compound 1c fumarate (re-prepared Fumarate Type A).
FIG. 13 depicts TGA and DSC thermograms for Compound 1c fumarate (re-prepared Fumarate Type A).
FIG. 14 is an ¹H NMR spectrum of Compound 1c fumarate (re-prepared Fumarate Type A).
FIG. 15 is a collection of XRPD patterns for samples of a compound of Compound 2.

### DETAILED DESCRIPTION

The disclosed synthetic methods comprise several improvements over the known method of RESCA synthesis (WO 2016/065435). The number of required steps has been reduced, less expensive reagents are used, and/or the yield has been improved compared to previously reported methods. In some embodiments, the use of column chromatography has been eliminated in some or all of the steps of the synthesis. In some embodiments, the synthesis retains some or all of the aforementioned benefits when the method is performed at a larger scale than the previously reported method.

The disclosed method uses a different synthetic route than the previously known method of RESCA synthesis. The first step of the improved method is to synthesize a compound of Formula (Ic): 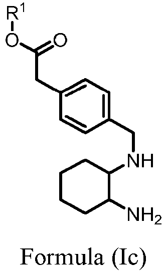 or a salt thereof, which provides at least one point to distinguish the method disclosed herein from the previous method. The improved synthetic route eliminates the need for a palladium catalyst. In some embodiments (*e.g*., Example 3, shown below), the products of some steps can be crystallized to eliminate the need for more complex purification of the intermediates.

### I. Definitions

As used herein, the term "C₁-Cₓ alkyl" alone or in combination means a straight-chain or branched alkyl moiety having 1 to x carbon atoms, wherein x is an integer. Accordingly, the term "C₁-C₆ alkyl" alone or in combination means a straight-chain or branched alkyl moiety having 1 to 6 carbon atoms. If present at the end of a molecule, some examples of straight-chain and branched C₁-C₆ alkyl groups are, without limitation, methyl, ethyl, n-propyl, isopropyl, n-butyl, isobutyl, sec-butyl, tert-butyl, n-pentyl, 2-methylbutyl, 2,2-dimethylpropyl, n-hexyl, 2-methylpenyl, 3-methylpentyl, 2,2-dimethylbutyl, 2,3-dimethylbutyl, and 3,3-dimethylpropyl. When two moieties of a molecule are linked by the C₁-C₆ alkyl group (*i.e.* the C₁-C₆ comprises a bivalent "alkylene" moiety), then examples include, without limitation, -CH₂-, -CH₂CH₂-, -CH(CH₃)-, -CH₂CH₂CH₂-, -CH(C₂H₅)-, and -C(CH₃)₂-.

As used herein, "halogen" refers to fluorine, chlorine, bromine, or iodine. The term "halo" refers to a halogen substituent, *i.e.* fluoro, chloro, bromo, or iodo.

As used herein, the term "halogenated hydrocarbon" refers to a hydrocarbon compound or moiety that is substituted at least once by a halogen moiety. Some halogenated hydrocarbons are useful as solvents for chemical synthesis. The term "halogenated hydrocarbon" includes, for example and without limitation, dichloromethane (also referred to as CH₂Cl₂ or DCM) and chloroform (also referred to as CHCl₃).

As used herein, the term "equivalent" means a molar equivalent unless otherwise specified.

As used herein, the term "chiral" refers to molecules that cannot be superimposed on their mirror image, while the term "achiral" refers to molecules that can be superimposed on their mirror image.

As used herein, the term "stereoisomers" refers to compound which have identical chemical formula (*i.e.* the same number of each type of atom), but differ with regard to the arrangement of the atoms or groups in physical space.

As used herein, the term "diastereomer" refers to a stereoisomer with two or more centers of chirality and whose molecules are not mirror images of each other. Diastereomers can have different physical properties (*e.g*. melting point, boiling point, spectral property, and reactivity).

As used herein, the term "enantiomer" refers to a molecule that is chiral and is a mirror image of another molecule. Two molecules that are mirror images of each other are "enantiomers." Enantiomers cannot be superimposed on each other.

Stereochemical definitions and conventions used herein generally follow S. P. Parker, Ed., McGraw-Hill Dictionary of Chemical Terms (1984) McGraw-Hill Book Company, New York; and Eliel, E. and Wilen, s., "Stereochemistry of Organic Compounds", John Wiley & Sons, Inc., New York, 1994. The compounds in the present application may contain asymmetric or chiral centers, and therefore exist as different stereoisomers. It is intended that all stereoisomers of the compounds recited herein, including but not limited to diastereomers, enantiomers, atropisomers, as well as mixtures thereof (*e.g*. racemic mixtures), are included in the application.

One or more compounds of the present application may exist in unsolvated as well as solvated forms with pharmaceutically acceptable solvents such as water, ethanol, and the like, and it is intended that the present application embraces both solvated and unsolvated forms. "Solvate" means a physical association of a compound of the present application with one or more solvent molecules. This physical association involves varying degrees of ionic and covalent bonding, including hydrogen bonding. In certain embodiments, the solvate will be capable of isolation, such as when one or more solvent molecules are incorporated in the crystal lattice of the crystalline solid. "Solvate" encompasses both solution-phase and isolatable solvates. Non-limiting examples of suitable solvates include solvates of ethanol, water, 1,4-dioxane, hydrochloric acid, and the like. "Hydrate" is a solvate wherein the solvent molecule is H₂O.

It is understood that embodiments described herein as "comprising" particular features imply that embodiments "consisting" and/or "consisting essentially of" those features have been contemplated.

Reference to "about" a value or parameter herein refers to the usual error range for the respective value readily known to the skilled person in this technical field. Reference to "about" a value or parameter herein includes (and describes) aspects that are directed to that value or parameter *per se.* For example, description referring to "about X" includes description of "X".

The term "and/or" as used herein a phrase such as "A and/or B" is intended to include both A and B; A or B; A (alone); and B (alone). Likewise, the term "and/or" as used herein a phrase such as "A, B, and/or C" is intended to encompass each of the following embodiments: A, B, and C; A, B, or C; A or C; A or B; B or C; A and C; A and B; B and C; A (alone); B (alone); and C (alone).

### II. Methods of synthesis

Provided herein are methods of synthesis for a compound that is capable of chelating a metal halide. The compound is a compound of Formula (I): 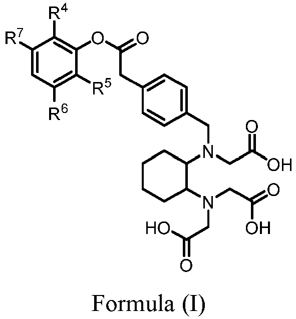 or a diastereomer thereof, a salt thereof, a solvate thereof, or a combination of any of the foregoing, wherein R^{4,} R⁵, R⁶ and R⁷ are each independently halo or hydrogen.

In some embodiments, the compound of Formula (I) is a compound of the structure: 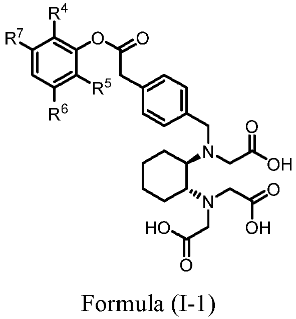 or a salt thereof, a solvate thereof, or a solvate of a salt thereof.

In some embodiments, the compound of Formula (I) is Compound 1: 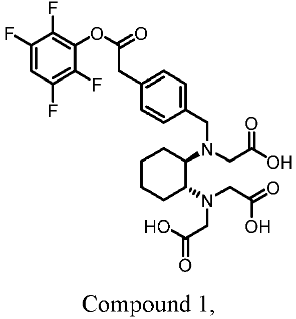 or a salt thereof, a solvate thereof, or a solvate of a salt thereof.

The methods described herein have chemical and/or commercial advantages over the previously disclosed methods of synthesis. In some embodiments, the yield of the present reactions is greater than reported the yields of Compound 1 or Compound 2 (depicted below) of previously known methods (*e.g*., WO 2016/065435). In some embodiments, the methods described herein provide products in high yields. In some embodiments, the yield is at least about 20%, at least about 30%, at least about 40%, at least about 50%, at least about 60%, at least about 70%, at least about 80%, at least about 90%, at least about 95%, at least about 98%, or at least about 99%. In some embodiments, the yield is between about 20% and about 30%, about 40%, about 50%, about 60%, about 70%, about 80%, about 90%, about 95%, about 98%, about 99%, or about 100%. In some embodiments, the yield is between about 30% and about 40%, about 50%, about 60%, about 70%, about 80%, about 90%, about 95%, about 98%, about 99%, or about 100%. In some embodiments, the yield is between about 40% and about 50%, about 60%, about 70%, about 80%, about 90%, about 95%, about 98%, about 99%, or about 100%. In some embodiments, the yield is between about 50% and about 60% about 70%, about 80%, about 90%, about 95%, about 98%, about 99%, or about 100%. In some embodiments, the yield is between about 60% and about 70%, about 80%, about 90%, about 95%, about 98%, about 99%, or about 100%. In some embodiments, the yield is between about 70% and about 80%, about 90%, about 95%, about 98%, about 99%, and about 100%. In some embodiments, the yield is between about 80% and about 90%, about 95%, about 98%, about 99%, or about 100%. In some embodiments, the yield is between about 90% and about 95%, about 98%, about 99%, or about 100%. In some embodiments, the yield is between about 95% and about 98%, about 99%, or about 100%. In some embodiments, the yield is between about 98% and about 99% or about 100%. In some embodiments, the yield is between about 99% and about 100%. In particular embodiments, the yield is between about 20% and about 30%. In other particular embodiments, the yield is about 50% to about 70%, such as about, in percent, 51, 52, 53, 54, 55, 56, 57, 58, 59, 60, 61, 62, 63, 64, 65, 66, 67, 68, 69, and 70.

In some embodiments, the method provides a product with greater purity than the previously disclosed methods. In some embodiments, the method provides a composition comprising a single diastereomer of Formula (I) (e.g., Compound 1 or Compound 2) having a purity of at least 80%, at least 90%, at least 95%, at least 96%, at least 97%, at least 98%, or at least 99%. In some embodiments, the purity is about 97.5 A%, 98 A%, and 99 A% (area %) as determined using high-performance liquid chromatography (HPLC).

In some embodiments, the reagents used in the present methods are cheaper than the reagents used in the previously disclosed methods. In some embodiments, the disclosed methods use reagents that are often less expensive and more friendly to the environment than those used in previously disclosed methods; however, importantly, the method does not require a palladium catalyst.

In some embodiments, a compound of Formula (I) (*e.g*., Compound 1 or Compound 2) can be prepared in a large scale. In some embodiments, "large scale" refers to the use of at least 100, 250, 500, 750, or 1000 grams of a starting material, intermediate, or reagent in any step of the process. In some embodiments, "large scale" refers to use of at least 1, 10, 25, 50, or 100 kg of a starting material, intermediate, or reagent in any step of the process.

In some embodiments, the present methods provide products with an improved yield and/or greater purity than the previously disclosed methods. In some embodiments, the present methods provide products with an improved yield and/or greater purity at a larger scale than previously known methods. Any of the yields, purities, and scales can be combined together as though each combination of a particular yield, particular purity, and particular scale were specifically and individually listed.

### A. First approach

Provided herein is a method of preparing a compound of Formula (I) 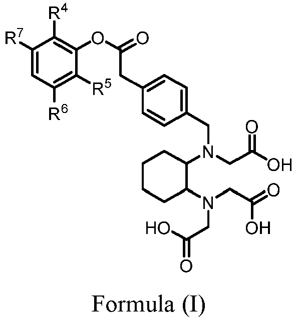 a diastereomer thereof, a salt thereof, a solvate thereof, or a combination of any of the foregoing, wherein R⁴, R⁵, R⁶, and R⁷ are each independently halo (*e.g*., F) or hydrogen, and wherein the method comprises steps (1-1) through (1-5). An exemplary synthetic scheme is shown in Scheme 1 of Example 1.

Step (1-1) comprises reacting a compound of Formula (Ia): 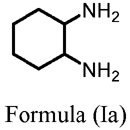 or a diastereomer thereof with a compound of Formula (Ib): 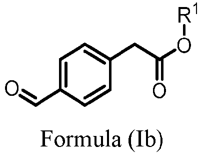 in a solvent and in the presence of a reducing agent to form a compound of Formula (Ic): 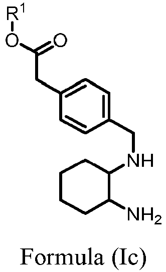 or a diastereomer thereof, wherein R¹ is C₁-C₆ alkyl.

In some embodiments, between about 0.5 to about 2 equivalents of the compound of Formula (Ib) are used compared to an amount of the compound of Formula (Ia). In some embodiments, about 1 equivalent is used.

In some embodiments, the compound of Formula (Ia) is a stereoisomer selected from the group consisting of

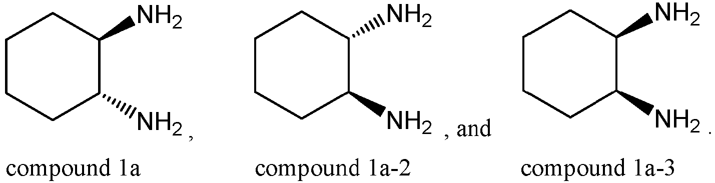

In some embodiments, the solvent of step (1-1) comprises an alcohol. In some embodiments, the solvent is methanol, ethanol, n-propanol, or isopropanol. In some embodiments, the solvent is ethanol.

In some embodiments, the reducing agent of step (1-1) comprises a hydride. In some embodiments, the reducing agent comprises NaBH(OAc)₃, NaBH₄CN, and NaBH₄. In some embodiments, the reducing agent is NaBH(OAc)₃. In some embodiments, 1 to 2 equivalents of the reducing agent are used compared to an amount of the compound of Formula (Ia) (*i.e*., the molar ratio of reducing agent to compound is between 1:1 and 2:1). In some embodiments, about 1.5 to about 2 equivalents of the reducing agent are used. In some embodiments, about 1.8 to about 2 equivalents of the reducing agent are used. In some embodiments, about 1.9 equivalents of the reducing agent are used.

In some embodiments, step (1-1) is carried out at a temperature maintained between about 20 °C to about 70 °C for a duration between about 1 to about 3 hours. In some embodiments, the temperature is maintained between about 30 °C to about 60 °C. In some embodiments, the temperature is between about 40 °C to about 50 °C. In some embodiments, the duration is about 2 hours. In some embodiments, step (1-1) is carried out at a temperature maintained between about 40 °C to about 50 °C for a duration of about 2 hours.

In some embodiments, step (1-1) further comprises purifying the compound of Formula (Ic). In some embodiments, the purification uses a silica gel column. In some embodiments, the purification uses a solvent mixture. In some embodiments, the solvent mixture comprises dichloromethane (DCM) and methanol (MeOH). In some embodiments, the HPLC comprises varying a ratio of DCM to MeOH from 100:0 to 30:1 (v/v) over time. In some embodiments, the MeOH comprises NH₃. In some embodiments, the NH₃ is present in the MeOH at a concentration of 7M.

In some embodiments, step (1-1) does not include purification of the compound of Formula (Ic).

In some embodiments, the compound of Formula (Ic) is a compound of one of the Formula below:

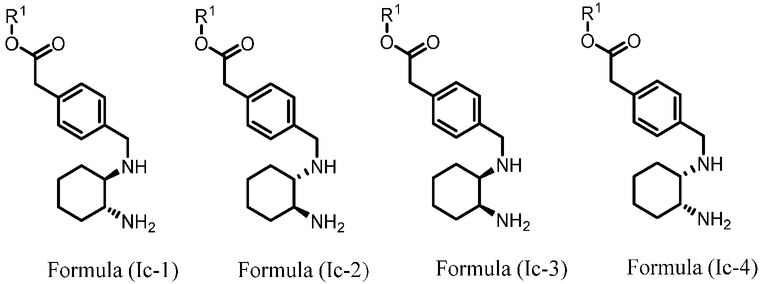

Step (1-2) comprises reacting the compound of Formula (Ic), or a diastereomer thereof, with a compound of Formula (Id) 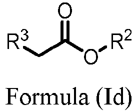
in the presence of a base and a solvent,
to form a compound of Formula (Ie): 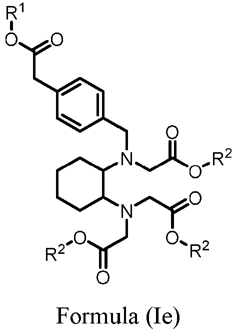
or a diastereomer thereof, wherein
R¹ and R² are each independently C₁-C₆ alkyl; and
R³ is halo.

In some embodiments, between about 2 to about 5 equivalents of the compound of Formula (Id) are used compared to an amount of the compound of Formula (Ic). In some embodiments, between about 3 to about 4 equivalents are used. In some embodiments, 3.5 equivalents are used.

In some embodiments, the solvent of step (1-2) comprises a polar organic solvent. In some embodiments, the solvent comprises CH₃CN, tetrahydrofuran (THF), MeTHF, acetone, methylethylketone, isopropylacetate, N,N-dimethylformamide (DMF), N-methyl-2-pyrrolidone (NMP), or dimethyl sulfoxide (DMSO). In some embodiments, the solvent is CH₃CN.

In some embodiments, the base of step (1-2) is an organic base. In some embodiments, the base is an amine base. In some embodiments, the base is triethylamine, tributylamine, N-methylmorpholine, N,N-diisopropylethylamine (DIPEA), or 1,8-Diazabicyclo[5.4.0]undec-7-ene. In some embodiments, the base is DIPEA. In some embodiments, between about 4 to about 12 equivalents of base are used compared to an amount of the compound of Formula (Ic). In some embodiments, between about 6 to about 10 equivalents of base are used. In some embodiments, between about 7 to about 9 equivalents of base are used. In some embodiments, about 8 equivalents of base are used.

In some embodiments, step (1-2) is performed in CH₃CN in the presence of DIPEA. In some such embodiments, about 8 equivalents of DIPEA are used.

In some embodiments, step (1-2) is carried out at a temperature maintained between about 20 °C to about 70 °C for a duration between about 12 to about 24 hours. In some embodiments, the temperature is maintained between about 30 °C to about 60 °C. In some embodiments, the temperature is between about 40 °C to about 50 °C. In some embodiments, the duration is between about 16 and about 20 hours. In some embodiments, the duration is about 18 hours. In some embodiments, step (1-2) is carried out at a temperature maintained between about 40 °C to about 50 °C for a duration of about 18 hours.

In some embodiments, step (1-2) further comprises purifying the compound of Formula (Ie). In some embodiments, step (1-2) further comprises purifying the compound of Formula (Ie) by column chromatography. In some embodiments, the purification uses a silica gel column. In some embodiments, the purification uses a solvent mixture comprising ethyl acetate and n-heptane. In some embodiments, the purification comprises varying a ratio of ethyl acetate to n-heptane from 100:0 to 1:5 (v/v) over time.

In some embodiments, step (1-2) does not include purification of the compound of Formula (Ie).

In some embodiments, the compound of Formula (Ie) is a compound of one of the Formula below:

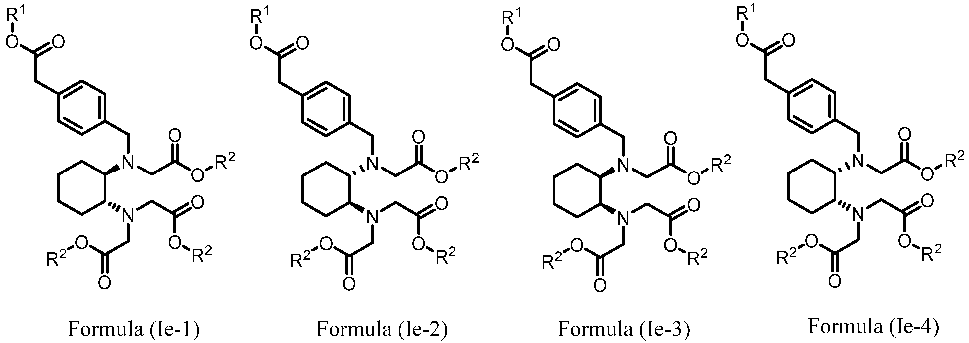

Step (1-3) comprises reacting the compound of Formula (Ie), or a diastereomer thereof, with a base in a solvent to form a compound of Formula (If): 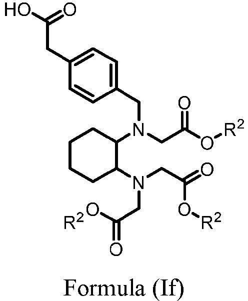 or a diastereomer thereof, wherein each R² is C₁-C₆ alkyl.

In some embodiments, the solvent of step (1-3) comprises water. In some embodiments, the solvent comprises an organic solvent. In some embodiments, the solvent is a mixture of water and an organic solvent. In some embodiments, the solvent comprises tetrahydrofuran (THF), MeTHF, methyl tertiary-butyl ether, cyclopentyl methyl ether, dichloromethane, and isopropyl acetate. In some embodiments, the solvent comprises THF. In some embodiments, the solvent is a mixture of water and THF. In some embodiments, a ratio of organic solvent to water is used. In some embodiments, the ratio of organic solvent to water is between about 4:1 and 1: 1. In some embodiments, the ratio is between about 10:3 and about 6:3. In some embodiments, the ratio is about 8:3. All of the ratios in the above embodiments are equally applicable to embodiments wherein the ratio is a ratio of THF to water.

In some embodiments, the base of step (1-3) is a strong base. In some embodiments, the base is KOH. In some embodiments, about 1 to about 3 equivalents of base are used compared to an amount of the compound of Formula (Ie). In some embodiments, about 2 equivalents of base are used.

In some embodiments, step (1-3) is performed in a mixture of water and THF in the presence of KOH. In some such embodiments, about 2 equivalents of KOH are used.

In some embodiments, the compound of Formula (If) is a compound of one of the Formula below:

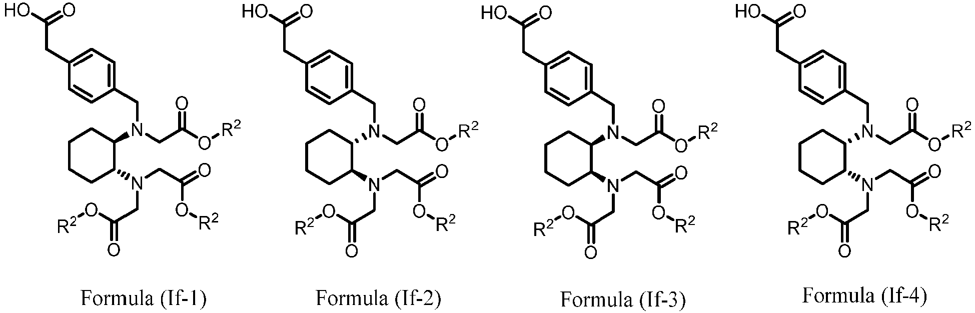

In some embodiments, step (1-3) is carried out at a temperature maintained between about -20 °C to about 30 °C for a duration between about 18 to about 30 hours. In some embodiments, the temperature is maintained between about -10 °C to about 20 °C. In some embodiments, the temperature is between about 0 °C to about 10 °C. In some embodiments, the duration is between about 16 to about 20 hours. In some embodiments, the duration is about 18 hours. In some embodiments, step (1-3) is carried out at a temperature maintained between about 0 °C to about 10 °C for a duration of about 18 hours.

Step (1-4) comprises reacting the compound of Formula (If), or a diastereomer thereof, with a compound of Formula (Ig): 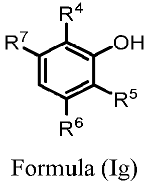
in a solvent and in the presence of a carboxyl activating reagent, to form a compound of Formula (Ih): 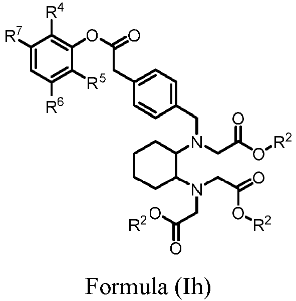
or a diastereomer thereof, wherein
each R² is independently C₁-C₆ alkyl; and
R⁴, R⁵, R⁶, and R⁷ are each independently halo or hydrogen.

In some embodiments, the compound of Formula (Ih) is a compound of one of the Formula below:

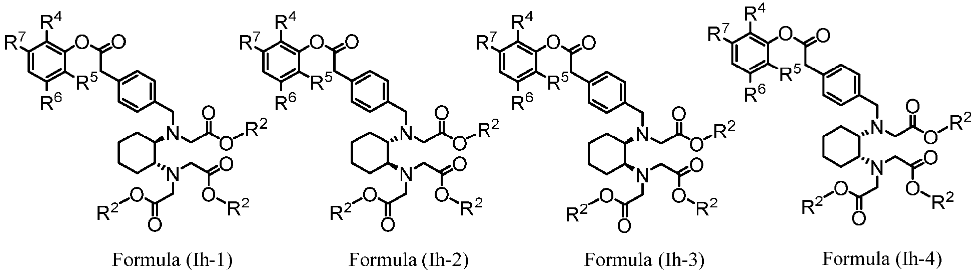

In some embodiments, between about 0.8 and about 1.6 equivalents of the compound of Formula (Ig) are used compared to an amount of the compound of Formula (If). In some embodiments, between about 1.0 and about 1.4 equivalents are used. In some embodiments, about 1.2 equivalents are used.

In some embodiments, the solvent of step (1-4) is a halogenated hydrocarbon. In some embodiments, the solvent is selected from the group consisting of 1,2-dichloroethane, CH₂Cl₂ and CHCl₃. In some embodiments, the solvent is CH₂Cl₂ (DCM or dichloromethane).

In some embodiments, the carboxyl activating agent of step (1-4) comprises a carbodiimide. In some embodiments, the carboxyl activating agent is 1-ethyl-3-(3-dimethylaminopropyl)-carbodiimide (EDCI).

In some embodiments, step (1-4) is carried out in DCM in the presence of EDCI.

In some embodiments, step (1-4) is carried out at a temperature maintained between about 0 °C to about 50 °C for a duration between about 8 to about 24 hours. In some embodiments, the temperature is maintained between about 10 °C to about 40 °C. In some embodiments, the temperature is between about 20 °C to about 30 °C. In some embodiments, the duration is between about 12 to about 20 hours. In some embodiments, the duration is about 16 hours. In some embodiments, step (1-4) is carried out at a temperature maintained between about 20 °C to about 30 °C for a duration of about 10 hours. In some embodiments, step (1-4) is carried out at a temperature maintained between about 20 °C to about 30 °C for a duration of about 16 hours.

Step (1-5) comprises reacting the compound of Formula (Ih), or a diastereomer thereof, with an acid to form a compound of Formula (I) or a diastereomer thereof. In some embodiments, the acid comprises HCl, HBr, H₂SO₄, methanesulfonic acid, p-toluenesulfonic acid, or trifluoroacetic acid. In some embodiments, the acid is trifluoroacetic acid.

In some embodiments, step (1-5) further comprises purifying the compound of Formula (I). In some embodiments, step (1-5) further comprises purifying the compound of Formula (I) by preparative high performance liquid chromatography (HPLC). In some embodiments, the HPLC is carried out with a 98:2 ratio of an aqueous solution of 0.01% TFA to acetonitrile.

In some embodiments, step (1-5) does not include purification of the compound of Formula (I).

In some embodiments, step (1-5) is carried out at a temperature maintained between about 0 °C to about 50 °C for a duration between about 8 to about 24 hours. In some embodiments, the temperature is maintained between about 10 °C to about 40 °C. In some embodiments, the temperature is between about 20 °C to about 30 °C. In some embodiments, the duration is between about 12 to 20 hours. In some embodiments, the duration is about 16 hours. In some embodiments, step (1-5) is carried out at a temperature maintained between about 20 °C to about 30 °C for a duration of about 16 hours.

In some embodiments, the compound of Formula (I) is a compound of one of the Formula below:

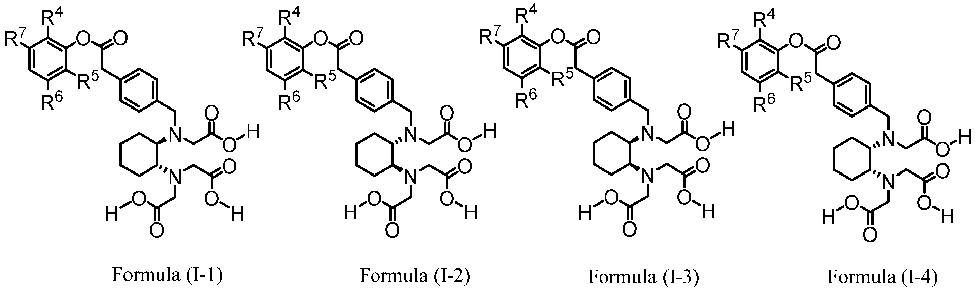

### B. Second approach

There is also provided a method of preparing a compound of Formula (I): 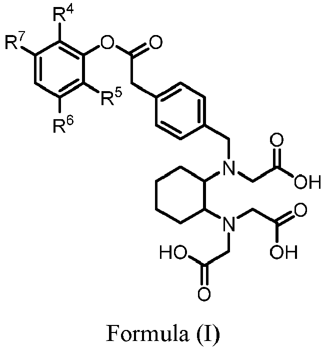 a diastereomer thereof, a salt thereof, a solvate thereof, or a combination of any of the foregoing, wherein R⁴, R⁵, R⁶, and R⁷ are each independently halo (*e.g*., F) or hydrogen, wherein the method comprises steps (2-1) through (2-6). An exemplary synthetic scheme is shown in Scheme 2 of Example 3. One advantage of the Second Approach is that crystallization steps eliminate the need for expensive and time consuming purification steps, such as silica gel chromatography.

Step (2-1) comprises reacting a compound of Formula (Ia): 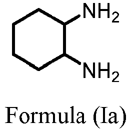 or a diastereomer thereof with a compound of Formula (Ib): 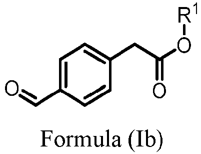 in a solvent and in the presence of a reducing agent to form a compound of Formula (Ic): 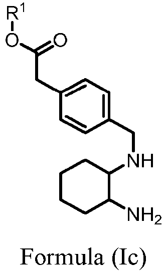 or a diastereomer thereof, wherein R¹ is C₁-C₆ alkyl.

In some embodiments, about 0.5 to about 3 equivalents of the compound of Formula (Ia) are used compared to an amount of the compound of Formula (Ib). In some embodiments, about 1 to about 2 equivalents are used. In some embodiments, about 1.3 equivalents are used.

In some embodiments, the compound of Formula (Ia) comprises, consists of, or consists essentially of one stereoisomer. In some embodiments, the compound of Formula (Ia) is a mixture of stereoisomers. In some embodiments, the compound of Formula (Ia) is a racemic mixture. In some embodiments, the compound of Formula (Ia) is selected from the group consisting of 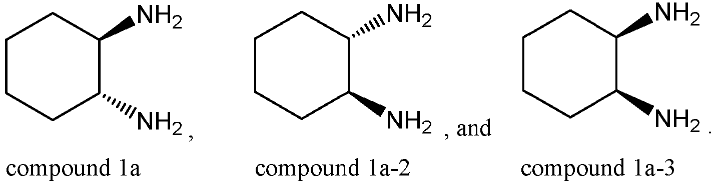 In some embodiments, the compound of Formula (Ia) is a mixture comprising two or more of the three stereoisomers shown above.

In some embodiments, the solvent of step (2-1) comprises an alcohol. In some embodiments, the solvent is methanol, ethanol, n-propanol, or isopropanol. In some embodiments, the solvent is ethanol.

In some embodiments, the reducing agent of step (2-1) comprises a hydride. In some embodiments, the reducing agent comprises NaBH(OAc)₃. In some embodiments, the reducing agent is NaBH(OAc)₃. In some embodiments, about 1 to about 5 equivalents of hydride are used compared to an amount of the compound of Formula (Ib). In some embodiments, about 2 to about 4 equivalents are used. In some embodiments, about 3 equivalents are used.

In some embodiments, step (2-1) is performed in ethanol in the presence of NaBH(OAc)₃. In some embodiments, 3 equivalents of NaBH(OAc)₃ are used.

In some embodiments, step (2-1) is carried out at a temperature maintained between about 20 °C to about 65 °C for a duration between about 1 to about 3 hours. In some embodiments, the temperature is maintained between about 30 °C to about 55 °C. In some embodiments, the temperature is between about 40 °C to about 45 °C. In some embodiments, the duration is about 2 hours. In some embodiments, step (2-1) is carried out at a temperature maintained between about 40 °C to about 45 °C for a duration of about 2 hours.

In some embodiments, the compound of Formula (Ic) is a compound of one of the Formula below:

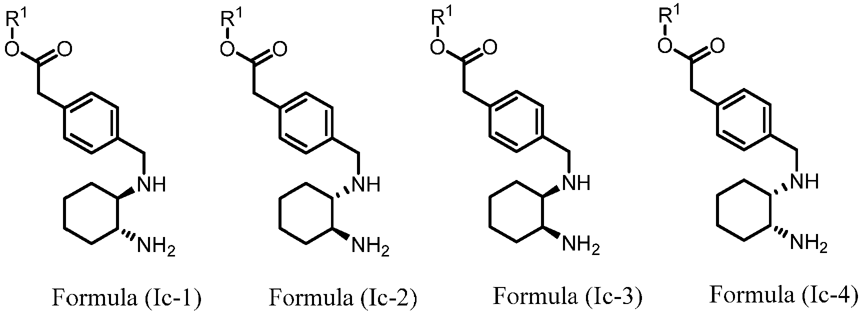

Step (2-2) comprises reacting a compound of Formula (Ic), or a diastereomer thereof, with an acid to produce a salt of Formula (Ic) or a diastereomer thereof. In some embodiments, the acid is an organic acid. In some embodiments, the acid is selected from the group consisting of fumaric acid, L-tartaric acid, and orotic acid.

In some embodiments, the acid is orotic acid. In some embodiments, the salt of Formula (Ic) or diastereomer thereof has the following structure: 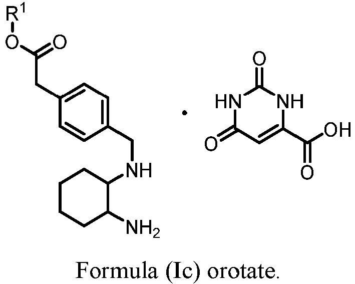 In some embodiments, the salt of Formula (Ic) is Compound 1c orotate: 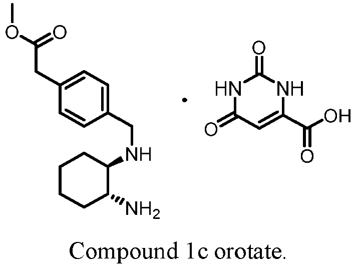 In some embodiments, step (2-2) comprises crystallizing the salt of Formula (Ic) or a diastereomer thereof. In some embodiments, step (2-2) comprises crystallizing the orotic acid salt of Formula (Ic) or a diastereomer thereof. In some embodiments, the salt of Formula (Ic) or diastereomer thereof is crystallized from acetone, 2-methyl-tetrahydrofuran, or an acetonitrile to water mixture (*e.g*., 19:1 CH₃CN to H₂O).

In some embodiments, the acid is fumaric acid. In some embodiments, the salt of Formula (Ic) or diastereomer thereof has the following structure: 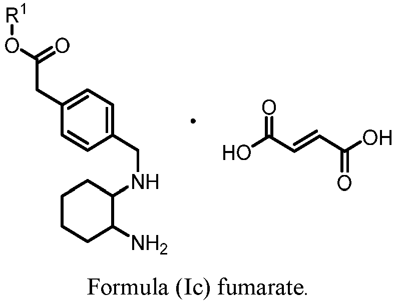 In some embodiments, the salt of Formula (Ic) is Compound 1c fumarate: 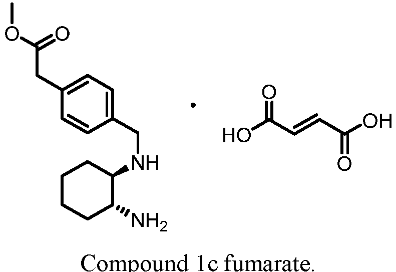 In some embodiments, step (2-2) further comprises crystallizing the salt of Formula (Ic), or a diastereomer thereof, as a salt of fumaric acid. In some embodiments, the salt of Formula (Ic) or diastereomer thereof is crystallized from acetone.

Step (2-3) comprises reacting the salt of Formula (Ic), or a diastereomer thereof, with a compound of Formula (Id): 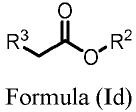
in the presence of a base and a solvent,
to form a compound of Formula (Ie): 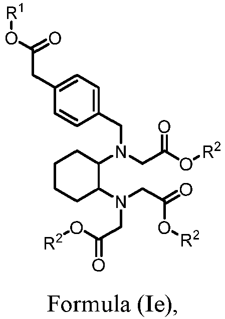
a diastereomer thereof, wherein
R¹ and R² are each independently C₁-C₆ alkyl; and
R³ is halo.

In some embodiments, between about 2 to about 5 equivalents of the compound of Formula (Id) are used compared to an amount of the salt of Formula (Ic). In some embodiments, between about 3 to about 4 equivalents are used. In some embodiments, about 3.5 equivalents are used.

In some embodiments, the base in step (2-3) comprises a carbonate salt. In some embodiments, the base comprises K₂CO₃. In some embodiments, the base is K₂CO₃. In some embodiments, about 1 to about 5 equivalents of base are used compared to an amount of the salt of Formula (Ic), diastereomer thereof. In some embodiments, about 2 to about 4 equivalents are used. In some embodiments, about 3 equivalents are used.

In some embodiments, the solvent in step (2-3) comprises water. In some embodiments, the solvent comprises an organic solvent. In some embodiments, the solvent comprises toluene. In some embodiments, the solvent is a mixture of water and an organic solvent. In some embodiments, the solvent is a mixture of water and toluene. In some embodiments, the water and the toluene are used in a ratio of between about 2:1 to about 1:2. In some embodiments, the ratio is about 1:1.

In some embodiments, step (2-3) is performed in a mixture of water and toluene in the presence of K₂CO₃. In some such embodiments, the water and toluene are in a ratio of about 1:1. In some such embodiments, about 3 equivalents of K₂CO₃ are used. In some such embodiments, the ratio of water to toluene is about 1:1 and about 3 equivalents of K₂CO₃ are used.

In some embodiments, step (2-3) is carried out at a temperature maintained between about 25 °C to about 65 °C for a duration between about 8 to about 24 hours. In some embodiments, the temperature is maintained between about 35 °C to about 55 °C. In some embodiments, the temperature is maintained at about 45 °C. In some embodiments, the duration is between about 12 and about 20 hours. In some embodiments, the duration is about 16 hours. In some embodiments, step (2-3) is carried out at a temperature maintained at about 45 °C for a duration between about 16 hours.

In some embodiments, the compound of Formula (Ie) is a compound of one of the Formula below:

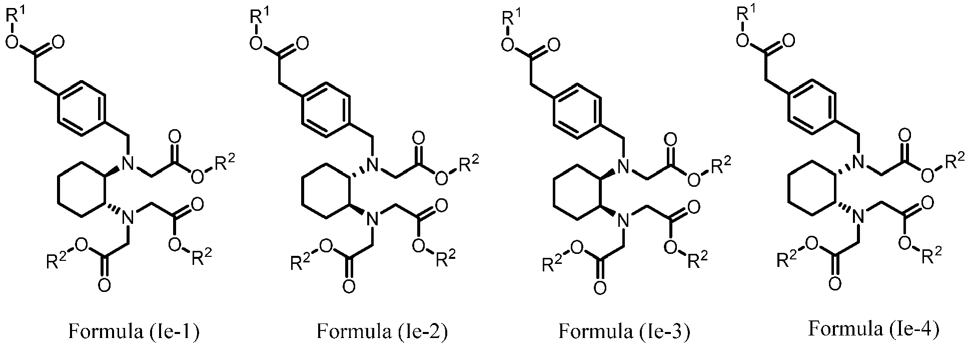

Step (2-4) comprises reacting a compound of Formula (Ie), or a diastereomer thereof, with a base in a solvent to form a compound of Formula (If): 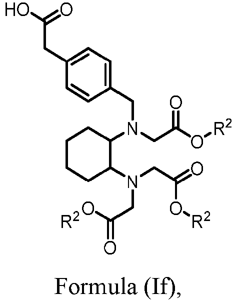 a diastereomer thereof, or a salt thereof.

In some embodiments, the base in step (2-4) is a strong base. In some embodiments, the base is KOH. In some embodiments, between about 1 and about 4 equivalents of KOH are used compared to an amount of a compound of Formula (Ie). In some embodiments, between about 2 and about 3 equivalents are used. In some embodiments, about 2.5 equivalents are used.

In some embodiments, the solvent in step (2-4) comprises THF. In some embodiments, the solvent comprises water. In some embodiments, the solvent is a mixture comprising water and THF.

In some embodiments, step (2-4) is performed in a mixture of water and THF in the presence of KOH. In some such embodiments, 2.5 equivalents of KOH are used.

In some embodiments, step (2-4) is carried out at a temperature maintained between about 0 °C and 5 °C for a duration between about 20 to about 60 hours. In some embodiments, the temperature maintained between about 0 °C and 5 °C for a duration between about 30 to about 40 hours. In some embodiments, the temperature maintained between about 0 °C and 5 °C for a duration of about 20 hours.

In some embodiments, step (2-4) provides the HCl salt of Formula (If) or a diastereomer thereof. In some embodiments, step (2-4) comprises crystallizing the salt of Formula (If) or diastereomer thereof. In some embodiments, the salt of Formula (If) has the structure: 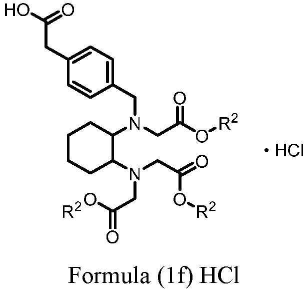 or is a diastereomer thereof.

In some embodiments, the HCl salt of Formula (If) has the structure:

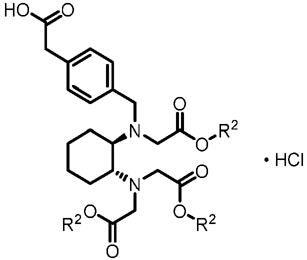

In some embodiments, the compound of Formula (If), or salt thereof, comprises a compound of one of the Formula below, or a salt thereof:

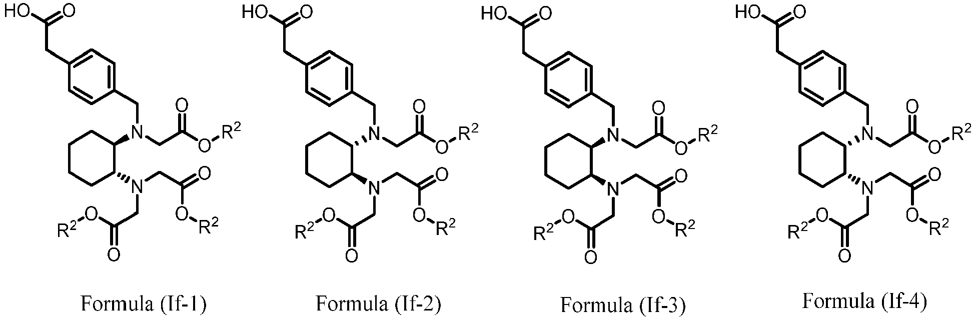

Step (2-5) comprises reacting the compound of Formula (If), diastereomer thereof, salt thereof, or diastereomer of a salt thereof with a compound of Formula (Ig): 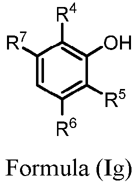 in a solvent and in the presence of a carboxyl activating reagent, to form a compound of Formula (Ih): 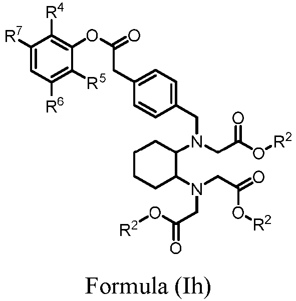 a diastereomer thereof, or a salt thereof.

In some embodiments, between about 1 to about 1.5 equivalents of the compound of Formula (Ig) are used compared to an amount of the compound of Formula (If). In some embodiments, about 1.2 equivalents are used.

In some embodiments, the solvent of step (2-5) is a halogenated hydrocarbon. In some embodiments, the solvent is selected from the group consisting of CH₂Cl₂ or CHCl₃. In some embodiments, the solvent is CH₂Cl₂.

In some embodiments, the carboxyl activating reagent of step (2-5) is 1-ethyl-3-(3-dimethylaminopropyl)carbodiimide (EDCI). In some embodiments, between about 1 to about 2 equivalents of EDCI are used compared to an amount of the compound of Formula (If). In some embodiments, about 1.5 equivalents are used.

In some embodiments, the step (2-5) is carried out in CH₂Cl₂ in the presence of EDCI.

In some embodiments, step (2-5) is carried out at a temperature maintained at between about 0 °C to about 5 °C for a duration between about 2 to about 4 hours. In some embodiments, the duration is about 3 hours. In some embodiments, step (2-5) is carried out at a temperature maintained between about 0 °C to about 5 °C for a duration of about 3 hours.

In some embodiments, the compound of Formula (Ih) is a compound of one of the Formula below:

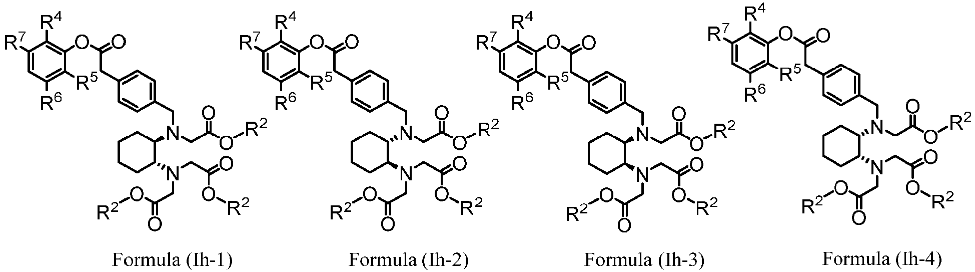

Step (2-6) comprises reacting a compound of Formula (Ih), or a diastereomer thereof, a salt thereof, or a diastereomer of a salt thereof, with an acid to form a compound of Formula (I) or a salt thereof. In some embodiments, step (2-6) comprises reacting a compound of Formula (Ih) with an acid to form a salt of a compound of Formula (I).

In some embodiments, the compound of Formula (I) is a compound of one of the Formula below, or a salt thereof:

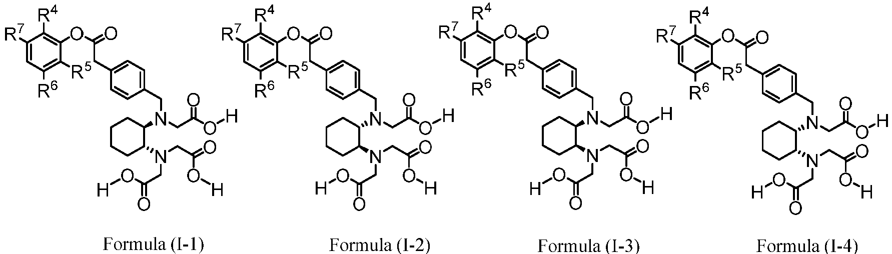

In some embodiments, the acid of step (2-6) is a mineral acid. In some embodiments, the acid of step (2-6) is hydrochloric acid. In some embodiments, the salt of Formula (I), or solvate thereof, is an HCl salt. In some embodiments, the salt of Formula (I), or solvate thereof, has the structure: 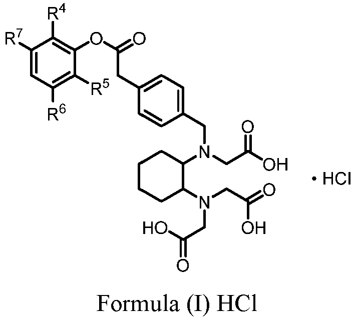 or is a diastereomer thereof. In some embodiments, the salt of Formula (I), or solvate thereof, has the structure:

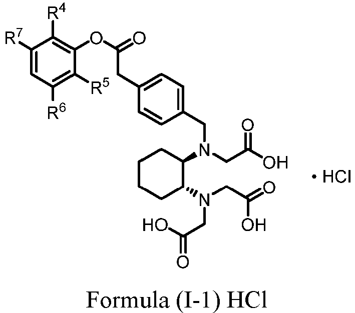

In some embodiments, step (2-6) is carried out in dioxane. In some embodiments, step (2-6) is carried out at a temperature maintained at between about 45 °C to about 70 °C for a duration between about 2 to about 4 hours. In some embodiments, the temperature is maintained at between about 55 °C to about 60 °C. In some embodiments, the duration is about 3 hours. In some embodiments, step (2-6) is carried out in dioxane at a temperature maintained between about 55 °C to about 60 °C for about 3 hours.

In some embodiments, the compound of Formula (I), or a salt, solvate, or salt of a solvate thereof, is Compound 2: 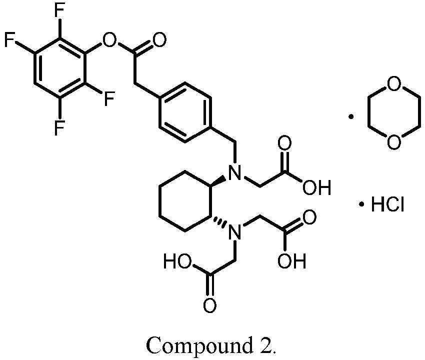 In some embodiments, the compound of Formula (I) is a diastereomer of Compound 2.

In some embodiments of any of the preceding methods (including the methods described in the "First approach" and "Second approach" subsections), R¹ is a straight-chain C₁-C₆ alkyl. In some embodiments, R¹ is C₁-C₃ alkyl. In some embodiments, R¹ is C₁-C₃ straight-chain alkyl. In some embodiments, R¹ is methyl, ethyl, isopropyl, n-propyl, t-butyl, or sec-butyl. In some embodiments, R¹ is methyl, ethyl, or isopropyl. Is some embodiments, R¹ is methyl or ethyl. In some embodiments, R¹ is methyl.

In some embodiments of any of the preceding methods (including the methods described in the "First approach" and "Second approach" subsections), R² is C₃-C₆ branched chain alkyl. In some embodiments, R² is methyl, ethyl, isopropyl, n-propyl, sec-butyl, or t-butyl. In some embodiments, R² is ethyl, isopropyl, or t-butyl. In some embodiments, R² is isopropyl, or t-butyl. In some embodiments, R² is t-butyl.

In some embodiments or any of the preceding methods (including the methods described in the "First approach" and "Second approach" subsections), R³ is F, Cl, or Br. In some embodiments, R³ is Cl or Br. In some embodiments, R³ is Br.

In embodiments of the preceding methods (including the methods described in the "First approach" and "Second approach" subsections), R⁴, R⁵, R⁶, and R⁷ are each independently halo or hydrogen. In some embodiments, R⁴, R⁵, R⁶, and R⁷ are each independently Cl or F. In some embodiments, R⁴, R⁵, R⁶, and R⁷ are each F.

### III. Compositions, labeled agents, and methods of use

Also disclosed herein are compositions comprising the compounds and intermediates disclosed herein. In particular, compositions of stereoisomers are contemplated for compounds of Formula (I), (Ic), (Ie), (If), and (Ih), including salts, solvates, and solvates of salts of any of the foregoing. Described herein is a composition comprising one or more diastereomers of compound of Formula (I), salts thereof, or solvates thereof: 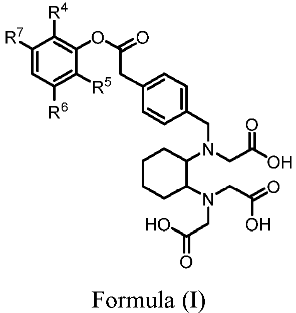 The compound of Formula (I) may be prepared using a method described in section II-A. The compound of Formula (I) may be prepared using a method described in section II-B. R⁴, R⁵, R⁶, and R⁷ may be each F.

The composition may comprise Compound 1, salts thereof, or solvates thereof: 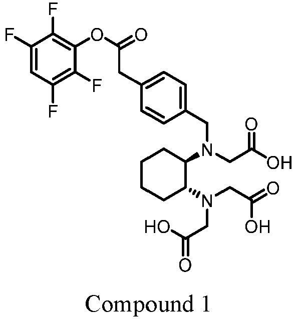 The composition may comprise, or consist of, or consist essentially of a single diastereomer. The composition may comprise at least about 80%, at least about 90%, at least about 95%, at least about 97%, or at least about 99% (weight/weight) of a trans diastereomer of Compound 1, or a salt, solvate, or solvate of a salt thereof. The composition may comprise at least about 80%, at least about 90%, at least about 95%, at least about 97%, or at least about 99% (weight/weight) of a trans diastereomer of Compound 2:

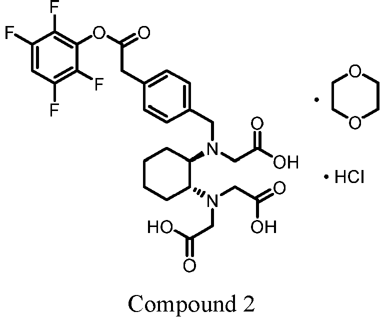

The composition may further comprise a detectable label. The detectable label may be a radioactive label, such as ¹⁸F. The detectable label may be part of a metal halide. The metal halide may be {AlF}²⁺. The metal halide may be {Al¹⁸F}²⁺. The label (*e.g*., metal halide) may form a complex with a linker. The linker may be a compound of Formula (I) (*e.g*., Compound 1 or Compound 2). The label may be chelated by the linker.

The composition may further comprise a polypeptide, wherein the compound of Formula (I) (*e.g*., Compound 1 or Compound 2) is conjugated to the polypeptide. The composition may comprise a polypeptide conjugated to a complex comprising the compound of Formula (I) (*e.g*., Compound 1 or Compound 2) and a metal halide (*e.g*., {Al¹⁸F}²⁺). The polypeptide may specifically bind a target molecule, such as a marker molecule. The polypeptide may be a marker-binding polypeptide or protein. The polypeptide may be an antibody or an antibody fragment. The antibody fragment may be selected from the group consisting of a VHH, a scFv, a minibody, a Fab, a Fab', and a Fv fragment. The polypeptide may be a non-antibody scaffold protein. The non-antibody scaffold protein may be selected from the group consisting of an affibody, an affilin, a knottin, a fibronectin, an anticalin, an atrimer, an avimer, an FN3 scaffold, a fynomer, a Kunitz domain, a pronectin, an OBody, and a DARPin.

Also described herein are compositions comprising marker-binding proteins, linkers, and labels. The composition may further comprise a marker-binding protein connected to a label by a linker. The linker may be Compound 1. The linker may be Compound 2. The label may be a metal halide. The metal halide may be {Al¹⁸F}²⁺. The linker may chelate the label. The linker may chelate a metal halide.

The term "antibody" herein is used in the broadest sense and encompasses various antibody structures, including but not limited to monoclonal antibodies, monovalent antibodies (*e.g*., one-armed antibodies), 4-chain antibodies (such as IgG antibodies), heavy chain only antibodies (HcAb), and antibody fragments thereof so long as they exhibit the desired antigen-binding activity. The terms "4-chain antibody" are used herein interchangeably to refer to an antibody or antigen-binding fragments having two heavy chains and two light chains.

"Antibody fragments" comprise a portion of an antibody, preferably the antigen binding or variable region of the antibody. Examples of antibody fragments include single-domain antibodies (including VHH antibodies), Fab, Fab', F(ab')₂, and Fv fragments; diabodies; linear antibodies (see U.S. Patent No. 5,641,870, Example 2; Zapata et al., Protein Eng. 8(10): 1057-1062 [1995]); single-chain antibody molecules; and multispecific antibodies formed from antibody fragments.

The composition may be suitable for administration to a subject. The composition may be suitable for intravenous administration. The subject may be a mammal. The subject may be a human.

Also described are methods of preparing a labeled agent using any one of the compounds described herein. Some relevant methods can be found in WO2021/046159 and Cleeren, F., Lecina, J., Bridoux, J. et al., Direct fluorine-18 labeling of heat-sensitive biomolecules for positron emission tomography imaging using the Al18F-RESCA method, Nat Protoc, 13, 2330-2347 (2018). The agent may be a marker-binding protein. Described herein is a method of preparing a labeled agent comprising a polypeptide and a ¹⁸F radionuclide, comprising: (a) contacting the polypeptide with any one of the compounds (*e.g*., Compound 1) or compositions prepared using the methods described herein under a condition that allows conjugation of the complex to the polypeptide to provide a conjugate, and (b) contacting the conjugate with an aluminum fluoride complex comprising ¹⁸F (*e.g*., {Al¹⁸F}²⁺) to provide the labeled agent. The compound may be conjugated to a lysine residue of the polypeptide. The conjugate may be contacted with the aluminum fluoride complex in the presence of one or more anti-oxidant compounds. The one or more anti-oxidant compounds may comprise methionine and/or N-acetyl-tryptophan. The conjugate may be contacted with the aluminum fluoride complex in the presence of methionine and N-acetyl-tryptophan. The method may comprise purifying the labeled agent from the reaction mixture comprising the conjugate and the aluminum fluoride by a desalting column. The desalting column may be equilibrated with a buffer comprising histidine, methionine, N-acetyl tryptophan, and/or sucrose. The desalting column may be equilibrated with a buffer comprising histidine, methionine, N-acetyl tryptophan, and sucrose.

Further described are labeled agents prepared using the methods described herein, and methods of using the labeled agents, for example, for *in vivo* imaging, diagnosis, prognosis, or monitoring. The labeled agent may be used for *in vivo* detection of a target molecule via specific binding of the polypeptide to the target molecule. The target molecule may be detected via at least one of: immuno PET (positron emission tomography), SPECT (single-photon emission computed tomography), MRI (magnetic resonance imaging), which is also known as NMR (nuclear magnetic resonance), near-infrared (NIR), or Cerenkov luminescence imaging (CLI).

The term "detecting" is intended to include determining the presence or absence of a substance or quantifying the amount of a substance (*e.g*., a target molecule). The term thus refers to the use of the materials, compositions, and methods of the present application for qualitative and quantitative determinations. In general, the particular technique used for detection is not critical for practice of the methods in the present application. For example, "detecting" according to the methods described herein may include: observing the presence or absence of a target molecule or a change in the levels of a target molecule. Detecting may include quantifying a change (increase or decrease) of any value between 10% and 90%, or of any value between 30% and 60%, or over 100%, when compared to a control. Detecting may include quantifying a change of any value between 2-fold to 10-fold, inclusive, or more e.g., 100-fold.

### IV. Compounds

Also disclosed herein are intermediates of the synthetic methods described herein. In some embodiments, the compound is a compound of Formula (Ie): 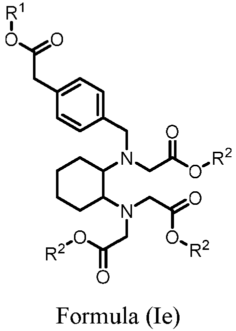
a diastereomer thereof, or a salt thereof,
wherein R¹ and R² are each independently C₁-C₆ alkyl.

In some embodiments, the compound of Formula (Ie), or a salt thereof, has a structure of one of the Formula below: 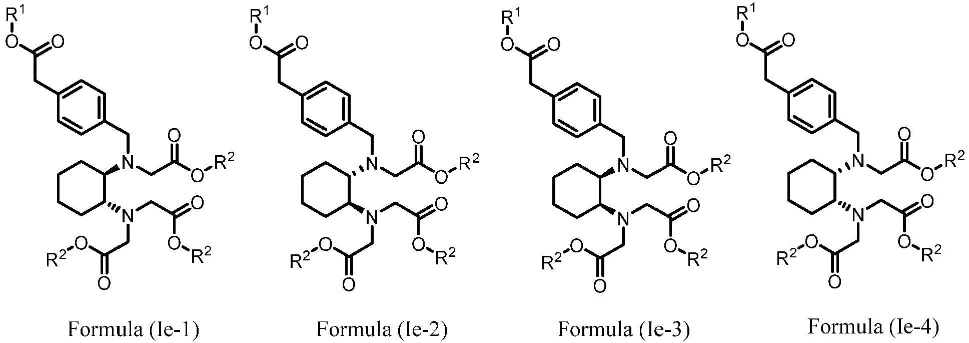 In some embodiments, the compound has the formula:

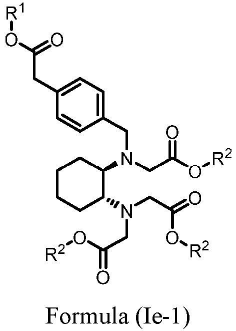

In some embodiments, the compound is a compound of Formula (Ic): 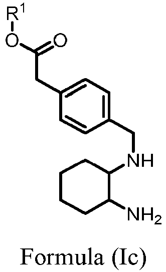 or a diastereomer or a salt thereof, wherein R¹ is C₁-C₆ alkyl.

In some embodiments, the compound of Formula (Ic), or a salt thereof, has a structure of one of the Formula below: 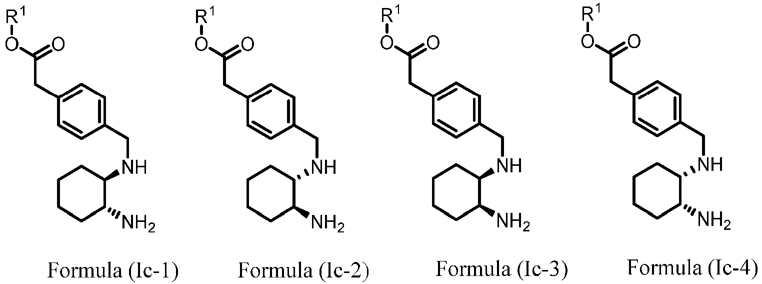 In some embodiments, the compound has the formula:

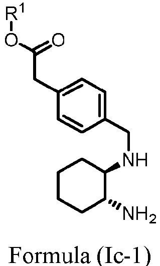

### Salts

Salts, or solvates thereof, of particular intermediates are disclosed.

In some embodiments, the salt is Compound 1c orotate: 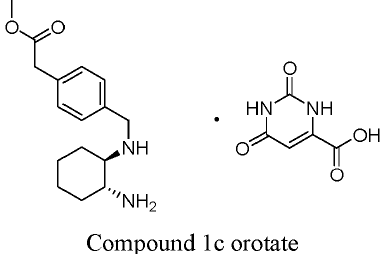 Compound 1c orotate is the orotic acid salt of a compound of Formula (Ic), wherein R¹ is methyl.

In some embodiments, the salt is Compound 1c fumarate: 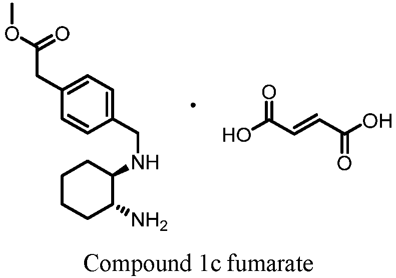 Compound 1c fumarate is the fumaric acid salt of a compound of Formula (Ic), wherein R¹ is methyl.

In some embodiments, the salt is Compound 2: 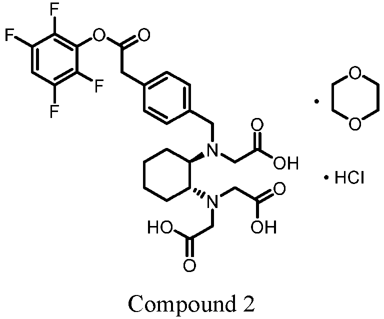 Compound 2 is a solvate of a salt of Compound 1.

In some embodiments, Compound 1c orotate has at least one of:
(a) an XRPD (X-ray powder diffraction) pattern with characteristic peaks at about 7.5° 2-Theta, about 13.9° 2-Theta, about 16.6° 2-Theta, about 23.4° 2-Theta, and about 25.6° 2-Theta;
(b) an XRPD pattern substantially the same as one of those shown in FIG. 1;
(c) a TGA (thermo-gravimetric analysis) thermogram showing a mass loss of about 3.3% between 20 °C and 150 °C;
(d) a TGA thermogram substantially the same as shown in FIG. 2;
(e) a DSC (differential scanning calorimetry) thermogram showing an endothermic peak at about 172.8 °C;
(f) a DSC thermogram substantially the same as shown in FIG. 2;
(g) an ¹H NMR (nuclear magnetic resonance) spectrum with a characteristic peak at about 5.68 ppm; or
(h) an ¹H NMR spectrum substantially the same as shown in FIG. 3.

In some embodiments, Compound 1c orotate has at least one of the following:
(a) an XRPD (X-Ray powder diffraction) pattern with characteristic peaks at about 7.2° 2-Theta, about 14.0° 2-Theta, about 15.2° 2-Theta, about 22.7° 2-Theta, and about 25.0° 2-Theta;
(b) an XRPD pattern substantially the same as one of those shown in FIG. 1;
(c) a TGA (thermo-gravimetric analysis) thermogram showing a mass loss of about 2.6% between 20 °C and 150 °C;
(d) a TGA thermogram substantially the same as shown in FIG. 4;
(e) a DSC (differential scanning calorimetry) thermogram showing an exothermic peak at about 161.7 °C and an endothermic peak at about 182.9 °C;
(f) a DSC thermogram substantially the same as shown in FIG. 4;
(g) an ¹H NMR (nuclear magnetic resonance) spectrum with a characteristic peak at about 5.69 ppm; and
(h) an ¹H NMR spectrum substantially the same as shown in FIG. 5.

In some embodiments, Compound 1c orotate has at least one of the following:
(a) an XRPD (X-Ray powder diffraction) pattern with characteristic peaks at about 7.3° 2-Theta, about 7.8° 2-Theta, about 9.7° 2-Theta, about 13.9° 2-Theta, about 16.7° 2-Theta, about 21.4° 2-Theta, about 23.4° 2-Theta;
(b) an XRPD pattern substantially the same as one of those shown in FIG.6;
(c) a TGA (thermo-gravimetric analysis) thermogram showing a mass loss of about 4.8% between 20 °C and 150 °C;
(d) a TGA thermogram substantially the same as shown in FIG. 7;
(e) a DSC (differential scanning calorimetry) thermogram showing an exothermic peak at about 178.1 °C and an endothermic peak at about 185.6 °C;
(f) a DSC thermogram substantially the same as shown in FIG. 7;
(g) an ¹H NMR (nuclear magnetic resonance) spectrum with a characteristic peak at about 5.68 ppm; and
(h) an ¹H NMR spectrum substantially the same as shown in FIG. 8.

Compound 1c fumarate has at least one of:
a) an XRPD (X-Ray powder diffraction) pattern with characteristic peaks at about 7.9° 2-Theta, about 15.8° 2-Theta, about 18.6° 2-Theta, about 19.3° 2-Theta, about 20.1° 2-Theta, about 21.0° 2-Theta, and about 24.7° 2-Theta;
(b) a TGA (thermo-gravimetric analysis) thermogram showing a mass loss of about 2.4% between 20 °C and 140 °C;
(c) a DSC (differential scanning calorimetry) thermogram showing an endothermic peak at about 154.7 °C; and
(d) an ¹H NMR (nuclear magnetic resonance) spectrum with a characteristic peak at about 6.39 ppm.

The crystallized salt of Compound 1c fumarate has at least one of:
(a) an XRPD (X-Ray powder diffraction) pattern with characteristic peaks at about 7.9° 2-Theta, about 15.8° 2-Theta, about 18.6° 2-Theta, about 19.3° 2-Theta, about 20.1° 2-Theta, about 21.0° 2-Theta, and about 24.7° 2-Theta;
(b) a TGA (thermo-gravimetric analysis) thermogram showing a mass loss of about 1.9% between 20 °C and 140 °C;
(c) a DSC (differential scanning calorimetry) thermogram showing an endothermic peak at about 156.2 °C; and
(d) an ¹H NMR (nuclear magnetic resonance) spectrum with a characteristic peak at about 6.39 ppm.

In some embodiments, Compound 2 has at least one of:
(a) an XRPD (X-Ray powder diffraction) pattern with characteristic peaks at about 6.8° 2-Theta, about 17.6° 2-Theta, about 20.3° 2-Theta, about 22.2° 2-Theta, and about 23.3° 2-Theta; and
(b) an XRPD pattern substantially the same as shown in FIG. 15.

### EXAMPLES

The following abbreviations are used throughout the Examples:
A% - area % based on HPLC analysis
HPLC - high performance liquid chromatography
¹H NMR - proton nuclear magnetic resonance
MeOH - methanol
EtOH - ethanol
EtOAc - ethyl acetate
DMSO - dimethylsulfoxide
NaBH(OAc)₃ - sodium triacetoxyborohydride
DIPEA - N,N-diisopropylethylamine
MTBE - methyl tertiary-butyl ether

### Example 1: Small-scale palladium free synthesis of Compound 1

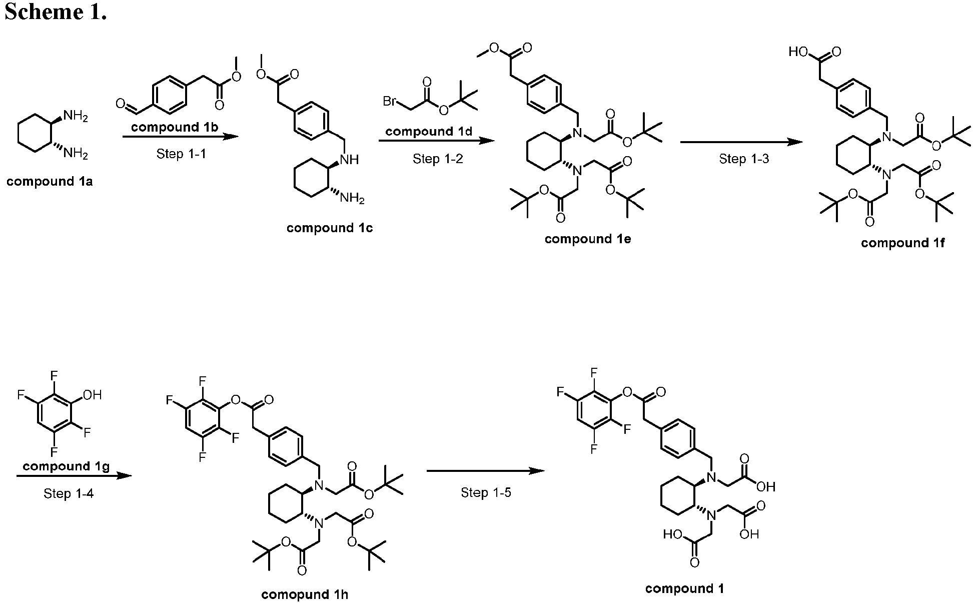

### Step 1a-1: Synthesis of compound 1c - methyl-2-(4-((((1R,2R)-2-aminocyclohexyl)-amino)methyl)phenyl)acetate

To a mixture of (1R,2R)-(-)-trans-1,2-diaminocyclohexane (compound 1a, 200 g, 1.75 mol) in EtOH (6 L, 30 mL/g) at 50 °C was added methyl 2-(4-formylphenyl)acetate (compound 1b, 310 g, 1.74 mol) then NaBH(OAc)₃ (730 g, 3.31 mol) portion-wise over 15 minutes maintaining temperature below 60 °C. The resulting mixture was stirred for 2 h before cooling to 20 °C, residual solids were removed by filtration and mother liquors were concentrated in vacuo then was purified by silica gel chromatography (3% MeOH 7 M NH₃ in CH₂Cl₂) to afford compound 1c (240 g, 0.87 mol, 50% yield, 96 A% HPLC). ¹H NMR (400 MHz, DMSO-*d*₆): 7.28 (d, *J =* 8.1 Hz, 2H), 7.19 (d, *J =* 8.1 Hz, 2H), 3.80 (d, *J =* 13.5 Hz, 1H), 3.64 (s, 2H), 3.61 (s, 3H), 3.59 (d, *J =* 13.6 Hz, 1H), 2.29 - 2.23 (m, 1H), 2.05 - 1.93 (m, 2H), 1.79 - 1.73 (m, 1H), 1.68 - 1.54 (m, 2H), 1.27 - 0.85 (m, 4H). ¹³C NMR (101 MHz, DMSO-*d*₆): δ 172.0, 140.8, 132.7, 129.4, 128.3, 63.5, 55.3, 52.0, 50.5, 40.4, 36.1, 31.2, 25.5, 25.4. HRMS *(m*/*z)* calcd for C₁₆H₂₄N₂O₂ [M+H]⁺ 277.1911, found 277.1912.

### Step 1a-2: Synthesis of compound 1e - di-tert-buty-2,2'-(((1R,2R)-2-((2-(tert-butoxy)-2-oxoethyl)(4-(2-methoxy-2-oxoethyl)benzyl)amino)cyclohexyl)azanediyl)diacetate

To a mixture of compound 1c (225 g, 0.81 mol) and DIPEA (842 g, 6.50 mol) in CH₃CN (2.3 L, 10 mL/g) at 50 °C was added compound 1d (556 g, 2.84 mol) and stirred for 18 h. CH₃CN was solvent switched with EtOAc (2.5 L, 11 mL/g) then organic solvent was washed with H₂O (3×400 mL, 2.1 mL/g), dried over Na₂SO₄, filtered, solvent removed in vacuo and purified by silica gel chromatography (20% EtOAc in n-heptane) to afford compound 1e (400 g, 0.65 mol, 79 % yield, 98 A% HPLC). ¹H NMR (400 MHz, DMSO-*d*₆): 7.33 (d, *J =* 8.1 Hz, 2H), 7.16 (d, *J =* 8.1 Hz, 2H), 4.50 (s, 2H), 3.90 (d, *J =* 13.5 Hz, 1H), 3.60 (s, 3H), 3.56 (d, *J =* 13.5 Hz, 1H), 3.41, 3.34 (ABq, *J =* 16.0 Hz, 4H), 3.27, 3.19 (ABq, *J* = 16.6 Hz, 2H), 2.75 - 2.65 (m, 1H), 2.56 - 2.50 (m, 1H), 1.95 - 1.87 (m, 2H), 1.66 - 1.56 (m, 2H), 1.39 (s, 18H), 1.35 (s, 9H), 1.30 - 1.27 (m, 1H), 1.06 - 0.97 (m, 3H).¹³C NMR (101 MHz, CDCl₃): δ 172.1, 171.6, 139.1, 132.3, 129.4, 128.9, 80.2, 63.6, 60.9, 54.3, 53.2, 52.7, 51.9, 40.9, 29.3, 28.4, 28.1, 25.8, 25.7. HRMS (*m*/*z*) calcd for C₃₄H₅₄N₂O₈ [M+H]⁺ 619.3953, found 619.3952.

### Step 1a-3: Synthesis of compound 1f - 2-(4-((((1R,2R)-2-(bis(2-(tert-butoxy)-2-oxoethyl)amino)cyclohexyl)(2-(tert-butoxy)-2-oxoethyl)amino)methyl)phenyl)acetic acid

To a solution of compound 1e (353 g, 0.60 mol) in THF (3 L, 8 mL/g) at 0 °C was added 1 M aqueous KOH (67.5 g, 1.2 mol in 1.2 L H₂O) while maintaining temperature below 10 °C. The resulting mixture was stirred for 24 h then pH was adjusted to 5-6 with 1 M aqueous HCl maintaining temperature below 10 °C. The solvent was switched from THF to CH₂Cl₂ (3.8 L, 10 mL/g), then the layers were separated, the organic solvent washed with 25% aqueous NaCl (1.85 L, 5 mL/g), dried over Na₂SO₄, filtered and concentrated in vacuo at 40 °C to afford compound 1f (327 g, 0.54 mol, 95% yield, 98 A% HPLC). ¹H NMR (400 MHz, CDCl₃): 9.10 (s, 1H), 7.38 (d, *J =* 7.9 Hz, 2H), 7.22 (d, *J =* 7.8 Hz, 2H), 4.09 (d, *J =* 13.4 Hz, 1H), 3.83 (d, *J =* 13.4 Hz, 1H), 3.61 (s, 2H), 3.48 (s, 2H), 3.35 (s, 4H), 2.76 - 2.65 (m, 2H), 2.14 (d, *J =* 12.2 Hz, 1H), 2.00 (d, *J =* 9.7 Hz, 1H), 1.73 - 1.64 (m, 2H), 1.43 (s, 18H), 1.40 (s, 9H), 1.28 - 1.18 (m 1H), 1.13 - 1.03 (m, 3H).¹³C NMR (101 MHz, CDCl₃): δ 172.6, 171.4, 170.5 165.2, 137.3, 131.7, 130.4, 127.9, 84.6, 82.2, 65.2, 60.9, 58.5, 55.9, 49.8, 41.5, 31.1, 28.1, 27.9, 26.0, 25.4, 24.5, 24.2. HRMS (*m*/*z*) calcd for C₃₃H₅₂N₂O₈ [M+H]⁺ 605.3796, found 605.3790.

### Step 1a-4: Synthesis of compound 1h - di-tert-butyl 2,2'-(((1R,2R)-2-((2-(tert-butoxy)-2-oxoethyl)(4-(2-oxo-2-(2,3,5,6-tetrafluorophenoxy)ethyl)benzyl)amino)cyclohexyl)-azanediyl)diacetate

To a mixture of compound 1f (180 g, 0.30 mol) and 2,3,5,6-tetrafluorophenol (59 g, 0.36 mol) in CH₂Cl₂ (1.8 L, 10 mL/g) at 25 °C was added EDCI (70 g, 0.45 mol) and stirred for 16 h. The mixture was cooled to 0 °C, washed with 1 M aqueous NaHCO₃ (3×1.8 L, 10 mL/g), and 25% aqueous NaCl solution (900 mL, 5 mL/g) the organic layer was dried over Na₂SO₄, filtered and the solvent was removed under vacuum to afford compound 1h (216 g, 96 % yield, 93 A% HPLC). ¹H NMR (400 MHz, CDCl₃): 7.39 (d, *J =* 7.8 Hz, 2H), 7.21 (d, *J =* 7.9 Hz, 2H), 6.95 - 6.86 (m, 1H), 3.97 (d, *J =* 13.6 Hz, 1H), 3.87 (s, 2H), 3.62 (d, *J =* 13.6 Hz, 1H), 3.42, 3.37 (ABq, *J =* 16.8 Hz, 4H), 3.30, 3.20 (ABq, *J =* 16.8 Hz, 2H), 2.70 - 2.60 (m, 1H), 2.52 - 2.45 (m, 1H), 2.01 - 1.93 (m, 2H), 1.65 - 1.57 (m, 2H), 1.36 (s, 18H), 1.34 (s, 9H), 1.21 - 0.94 (m, 4H). ¹³C NMR (101 MHz, CDCl₃): δ 172.0, 171.6, 167.5, 146.0 (dtd, *J =* 248.5, 11.9, 4.2 Hz), 140.6 (dddd, *J =* 250.8, 15.2, 4.6, 2.1 Hz), 140.0, 130.5, 129.7, 128.9, 103.2 (t, *J* = 22.8 Hz), 80.2, 63.6, 61.0, 54.2, 53.4, 53.1, 52.7, 39.9, 29.2, 28.4, 28.1, 25.8, 25.7. HRMS (*m*/*z*) calcd for C₃₉H₅₂F₄N₂O₈ [M+H]⁺ 753.3733, found 753.3744.

### Step 1a-5: Synthesis of compound 1 - 2,2'-(((1R,2R)-2-((carboxymethyl)(4-(2-oxo-2-(2,3,5,6-tetrafluorophenoxy)ethyl)benzyl)amino)cyclohexyl)azanediyl)diacetic acid

Compound 1h (210 g, 0.28 mol) was dissolved in TFA (2.1 L, 10 mL/g) at 20 °C and stirred for 4 h. The solution was concentrated under vacuum at 40 °C and purified by preparative HPLC to afford compound 1 (95 g, 59% yield, 0.16 mol, 99A% HPLC, 99% ee). ¹H NMR (400 MHz, CD₃OD): 7.67 (d, *J* = 8.2 Hz, 2H), 7.44 (d, *J =* 8.0, 2H), 7.43 - 7.34 (m, 1H), 4.59 (br s, 1H), 4.09 (s, 3H), 3.94 - 3.33 (br s, 3H), 3.52 (d, *J* = 16.4 Hz, 2H), 3.28 - 3.06 (m, 3H), 2.29 (d, *J* = 12.3 Hz, 1H), 2.19 (d, *J* = 10.5 Hz, 1H), 1.92 - 1.77 (m, 2H), 1.62 -1.40 (m, 1H), 1.38 - 1.19 (m, 3H). ¹³C NMR (101 MHz, CD₃OD): δ 172.2, 171.9, 169.7, 167.5, 146.1 (dtd, *J =* 247.5, 12.2, 2.8 Hz), 140.5 (ddm, *J =* 250, 16.2 Hz), 130.9, 130.7, 129.9, 129.4, 103.4 (t, *J =* 22.2 Hz), 62.4, 61.1, 50.9, 40.0, 38.9, 25.1, 24.2, 24.0. HRMS *(m*/*z)* calcd for C₂₇H₂₈F₄N₂O₈ [M+H]⁺ 585.1855, found 585.1854.

### Example 2: Large-scale palladium free synthesis of Compound 1

Scheme 1 applies to the synthesis below.

### Step 1b-1: Synthesis of compound 1c - methyl-2-(4-((((1R,2R)-2-aminocyclohexyl)-amino)methyl)phenyl)acetate

To a 100 L jacket reactor was added compound 1b (2.20 kg, 12.35 mol, 1 equiv), compound 1a (1.41 kg, 12.35 mol, 1 equiv) and EtOH (44 L, 20 mL/g). The mixture temperature was adjusted to 40-50 °C. NaBH(OAc)₃ (5.28 kg, 24.91 mol, 2 equiv) was added to the mixture at 40-50 °C in portions. Then the reaction mixture was stirred for 2 h at 40-50 °C. The reaction was monitored by HPLC (6 A% of compound 1b, 77 A% of compound 1c). The temperature of the reaction mixture was adjusted to 20-30 °C. Then the reaction mixture was filtered and the filter cake was washed with EtOH (5 L, 2.3 mL/g). The filtrate was concentrated to dryness under vacuum at below 45 °C. The crude oil product was purified by silica gel column chromatography with DCM/MeOH (7M NH₃) = 100/0 - 30/1 (v/v). The product-containing fractions were collected and concentrated to dryness under vacuum at below 45 °C. 1.75 kg of compound 1c was obtained as an oil with 95.4 A% HPLC purity in 51% yield.

### Step 1b-2: Synthesis of compound 1e - di-tert-buty-2,2'-(((1R,2R)-2-((2-(tert-butoxy)-2-oxoethyl)(4-(2-methoxy-2-oxoethyl)benzyl)amino)cyclohexyl)azanediyl)diacetate

To a 50 L jacket reactor was added compound 1c (1.70 kg, 6.15 mol, 1 equiv), CH₃CN (17 L, 10 mL/g) and diisopropylethylamine (DIPEA, 6.40 kg, 49.52 mol, 8.0 equiv). The temperature of the mixture was adjusted to 40-50 °C. Then compound 1d (4.20 kg, 21.53 mol, 3.5 equiv) was added into mixture at 40-50 °C in 3 portions in three hours. The reaction mixture was stirred for 18 h at 40-50°C. The reaction was monitored by HPLC (0 A% of compound 1c, 92 A% of compound 1e). The mixture was concentrated to dryness under vacuum at below 45 °C. Then ethyl acetate (17 L, 10 mL/g) was added into the mixture. The mixture was washed with H₂O (5 L, 3 mL/g) three times. The organic layer was separated and dried over Na₂SO₄ (2.50 kg). The mixture was then filtered and the filter cake was washed with ethyl acetate (4 L, 2.4 mL/g). The filtrates were combined and concentrated to dryness at below 45 °C under vacuum. The crude product was purified by silica gel column chromatography with ethyl acetate/n-Heptane = 0/100 - 1/5 (v/v). The product-containing fractions were collected and concentrated into dryness under vacuum. 2.75 kg of compound 1e was obtained as an oil with 97.4 A% HPLC purity in 72% yield.

### Step 1b-3: Synthesis of compound 1f - 2-(4-((((1R,2R)-2-(bis(2-(tert-butoxy)-2-oxoethyl)amino)cyclohexyl)(2-(tert-butoxy)-2-oxoethyl)amino)methyl)phenyl)acetic acid

To a 50 L jacket reactor was added compound 1e (2.60 kg, 4.20 mol, 1 equiv) and THF (26 L, 10 mL/g). The reaction mixture temperature was adjusted to 0-10 °C. Then aqueous KOH solution (8.6 mol, 2 equiv, 0.48 kg KOH in H₂O 8.4 L, 3 mL/g) was added into the reaction mixture at 0-10 °C in 4 portions for 4 hours. The reaction mixture was stirred for 24 h at 0-10 °C. The reaction was monitored by HPLC (1 A% of compound 1e, 95 A% of compound 1f). The reaction mixture was adjusted pH to 5-6 with aqueous 1 M HCl solution (10 kg) at 0-10 °C. Then the mixture was concentrated to remove THF at below 40 °C under vacuum. The reaction mixture was extracted with DCM (25 L, 9.5 mL/g) twice. The organic layers were combined and dried over Na₂SO₄ (2.50 kg), filtered over a silica gel pad (2.50 kg) and then the filter cake was washed with DCM (8 L, 3.1 mL/g). The filtrates were combined and concentrated to dryness at below 45 °C under vacuum. 2.20 kg of compound 1f was obtained with 98 A% HPLC purity in 87% yield as a light yellow solid.

### Step 1b-4: Synthesis of compound 1h - di-tert-butyl 2,2'-(((1R,2R)-2-((2-(tert-butoxy)-2-oxoethyl)(4-(2-oxo-2-(2,3,5,6-tetrafluorophenoxy)ethyl)benzyl)amino)cyclohexyl)-azanediyl)diacetate

To a 50 L jacket reactor was added compound 1f (2.00 kg, 3.31 mol, 1 equiv), compound 1g (0.72 kg, 4.34 mol, 1.3 equiv) and DCM (21 L, 10.5 mL/g). The reaction mixture temperature was adjusted to 20-30 °C. EDCI (0.81 kg, 4.23 mol, 1.3 equiv) was added into the reaction mixture in portions. The reaction mixture temperature was adjusted to 20-30 °C and stirred for 10 h. The reaction was monitored by HPLC (2 A% of compound 1f, 98 A% of compound 1h). The reaction mixture temperature was adjusted to 0-10 °C and then washed with 0.5 M aqueous NaHCO₃ solution (16 kg) five times at 0-10 °C. The two layers were separated and the organic layer was monitored by HPLC (0.3 A% of SM4, 93 A% of D). The organic layer was dried over Na₂SO₄ (2 kg), filtered over a silica gel pad (2 kg) and the cake was washed with DCM (17 L, 8.5 mL/g). The filtrates were combined and concentrated to about 4 kg at below 35 °C under vacuum to obtain crude compound 1h (2.49 kg) with 95 A% purity in 100% yield.

### Step 1b-5: Synthesis of compound 1 - 2,2'-(((1R,2R)-2-((carboxymethyl)(4-(2-oxo-2-(2,3,5,6-tetrafluorophenoxy)ethyl)benzyl)amino)cyclohexyl)azanediyl)diacetic acid

To a 50 L jacket reactor was added crude compound 1h (2.49 kg) and TFA (41 kg, 10 mL/g). The reaction mixture temperature was adjusted to 20-30 °C and stirred for 16 h. The reaction was monitored by HPLC (0 A% of compound 1h, 94 A% of compound 1). The reaction mixture was then concentrated at below 45 °C under vacuum. 6 kg of crude compound 1 as an oil (1.93 kg) was obtained with 91 A% HPLC purity.

The crude compound 1 was purified by prep-HPLC (Mobile phase A: 0.01% TFA aqueous solution (v/v); Mobile phase B: Acetonitrile) and the purified product was lyophilized to give compound 1 with 98 A% HPLC purity as a white solid in 51% yield over two steps.

(Note: The compound 1 in prep-HPLC fractions is not stable, and it needs to freeze immediately for lyophilization. The residual TFA in solid is 0.1 equiv to E)

### Example 3: Large-scale synthesis of compound 2

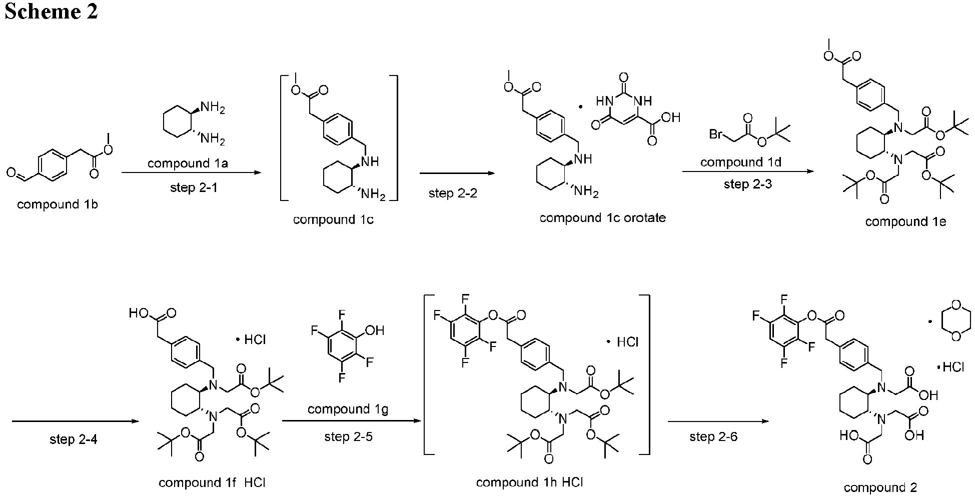

### Step 2-1: Synthesis of compound 1c - methyl-2-(4-((((1R,2R)-2-aminocyclohexyl)-amino)methyl)phenyl)acetate

To a 3 L jacket reactor, 50 g compound 1b and 1500 mL EtOH was added. The mixture was warmed to 45-50 °C. Then compound 1a (41.66 g ,1.3 equiv) was added. The mixture was stirred at 45-50 °C for 10 min. Then NaBH(OAc)₃ (178.42 g ,3.0 equiv) was added. The mixture was stirred at 45-50 °C for 2 h. The reaction was monitored by HPLC (77 A% of compound 1c, 8 A% of compound 1b). The reaction was filtered and washed with 150 mL EtOH. The filtrate was added 1000 mL water and concentrated at 30-35 °C to remove EtOH. Another 500 mL water and 500 mL MeTHF was added, and the mixture was stirred for 10 min and layers were separated. The aqueous phase was adjusted to pH 9.1 with NH₃•H₂O, and extracted with 250 mL DCM twice. The DCM layers were combined, dried over Na₂SO₄, and filtered to give 882 g compound 1c as a DCM solution in 66% corrected yield (89.3 A%, assay 5.78 wt%).

### Step 2-2: Synthesis of the orotic acid salt of compound 1c

500 mL MeCN was added to the above DCM solution of compound 1c. 2,4-dioxo-1*H*-pyrimidine-6-carboxylic acid (28.79 g ,1.0 equiv) was added to the solution. The reaction was stirred for 16 h at 20-25 °C. Then the mixture was filtered and the filter cake was washed with 100 mL MeCN. The filter cake was dried under vacuum at 30-35 °C for 20 h. 79.29 g of the orotic acid salt of compound 1c was obtained in 64% corrected yield over two steps from compound 2b (97.1 A% HPLC purity, assay 98.2 wt%). ¹H NMR (400MHz, DMSO-d6) δ = 7.36 (d, *J =*7.9 Hz, 2H), 7.18 (d, *J* =7.9 Hz, 2H), 5.80 (s, 1H), 3.86 (d, *J* =13.4 Hz, 1H), 3.66 - 3.57 (m, 6H), 2.78 (dt, *J* =3.9, 10.9 Hz, 1H), 2.39 (dt, *J* =3.7, 10.3 Hz, 1H), 2.13 (br d, *J* =11.7 Hz, 1H), 2.02 (br d, *J* =11.6 Hz, 1H), 1.66 (br d, *J* =9.0 Hz, 2H), 1.44 - 1.28 (m, 1H), 1.26 - 0.98 (m, 3H).

### Step 2-3: Synthesis of compound 1e - di-tert-buty-2,2'-(((1R,2R)-2-((2-(tert-butoxy)-2-oxoethyl)(4-(2-methoxy-2-oxoethyl)benzyl)amino)cyclohexyl)azanediyl)diacetate

To a 5 L jacket reactor were added the orotic acid salt of compound 1c (135.70 g, assay 95.8 wt%), 1300 mL toluene and 1300 mL water. Then K₂CO₃ (124.63 g, 3.0 eq.) was added and the mixture was stirred for 0.5 h at 20-25 °C. Compound 2d (205.22 g, 3.5 equiv) was added. The mixture was warmed to 40-45 °C and stirred for 16 h at 40-45 °C. The reaction was monitored by HPLC (93.8 A% of B, none of compound 1c remained). The reaction mixture was filtered to remove orotic acid and the cake was washed with 390 mL toluene. The layers were separated. The organic layer was washed with aqueous NaOH (1 equiv, 12.0 g NaOH in 1300 mL H₂O) at 20-25 °C for 0.5 h for 3 times to remove residual compound 2d. The organic layer was concentrated to dryness. 206.8 g of compound 1e was obtained as an oil with 96.6 A% HPLC purity and 81 wt% assay in 90% corrected yield. Residual toluene is 8.0 wt%, and residual compound 2d is 5.2 wt%. ¹H NMR (400MHz, DMSO-d6) δ = 7.34 (d, *J* =7.9 Hz, 2H), 7.21 - 7.12 (m, 2H), 3.91 (d, *J* =13.6 Hz, 1H), 3.68 - 3.53 (m, 6H), 3.47 - 3.31 (m, 4H), 3.24 (q, *J* =16.5 Hz, 2H), 2.75 - 2.63 (m, 1H), 2.01 - 1.83 (m, 2H), 1.60 (br d, *J* =6.2 Hz, 2H), 1.48 - 1.32 (m, 27H), 1.25 - 0.96 (m, 5H).

### Step 2-4: Synthesis of compound 1f hydrochloride - 2-(4-((((1R,2R)-2-(bis(2-(tert-butoxy)-2-oxoethyl)amino)cyclohexyl)(2-(tert-butoxy)-2-oxoethyl)amino)methyl)phenyl)acetic acid

To a 5 L jacket reactor, 185.19 g compound 1e (81 wt% assay by QNMR) and 1500 mL THF were added. The mixture was cooled to 0-5 °C. Then aqueous KOH (2.5 equiv 34 g KOH in 450 mL water) was slowly added to the reaction mixture at 0-5 °C. The reaction mixture was stirred at 0-5 °C for 40 h. The reaction was monitored by HPLC (95.2 A% of compound 1f, none of compound 1e remained). The mixture was adjusted to pH 2.0 with aq 1M HCl solution (about 700 mL, 4.5 v). The mixture was extracted with 1500 mL DCM. The organic layer was distilled and swapped with DCM twice (750 mL DCM each time). 509.4 g of compound 1f in DCM solution was obtained in 107% corrected yield. (Assay is 30.7 wt%, residual of THF is 5.5 wt%, residual of toluene is 1.1 wt%). 508.9 g of C in DCM solution (1.7 v DCM) was added in 2 L jacket reactor. 230 mL DCM (1.5 v) and 230 mL MTBE (1.5 v) were added. The mixture was stirred to a clear solution at 25 °C. 230 mL MTBE (1.5 v) was added to the mixture. 1.7 g seed of C was added. The mixture was stirred for 21 h at 25 °C. 1400 mL MTBE (9 v) was added over 8 h at 25 °C. The mixture was stirred at 25 °C for 40 h. The mixture was filtered and the filter cake was washed with 940 mL (DCM: MTBE = 1:4, v: v) for 3 times. The filter cake was dried at 25 °C for 24 h. 154.2 g of compound 1f hydrochloride with 98.0 A% HPLC purity and 90.2 wt% assay was obtained in 90% corrected yield from compound 1e. ¹H NMR (400MHz, CHLOROFORM-d) δ = 9.16 - 9.14 (m, 1H), 8.97 (br s, 1H), 7.65 - 7.58 (m, 2H), 7.36 - 7.26 (m, 2H), 4.73 - 4.55 (m, 2H), 4.41 - 4.07 (m, 1H), 4.00 - 3.81 (m, 1H), 3.78 - 3.65 (m, 2H), 3.66 - 3.33 (m, 2H), 3.25 - 3.03 (m, 2H), 2.81 - 2.50 (m, 2H), 2.20 - 1.70 (m, 3H), 1.60 - 1.33 (m, 28H), 1.26 - 1.12 (m, 2H)

### Step 2-5: Synthesis of compound 1h hydrochloride - di-tert-butyl 2,2'-(((1R,2R)-2-((2-(tert-butoxy)-2-oxoethyl)(4-(2-oxo-2-(2,3,5,6-tetrafluorophenoxy)ethyl)benzyl)amino)cyclohexyl)-azanediyl)diacetate

To a 1 L jacket reactor, 33.26 g of compound 1f hydrochloride (assay 90.2 wt%) and compound 1g (9.89 g, 1.2 equiv) were added. The mixture was degassed with N₂ for 3 times. 300 mL DCM was added to the mixture. The mixture was cooled to 0-5 °C under N₂ protection. EDCI (13.31 g, 1.4 equiv) was added. The mixture was stirred for 20 h at 0-5 °C. The reaction was monitored by HPLC (89.2 A% compound 1g, none of compound 1f remained). 300 mL water was added and the mixture was warmed to 20-25 °C. The layers were separated. KF of organic layer is 0.38%. The organic layer was recirculated through an Al₂O₃ filtration cartridge (Al₂O₃, 75.3 g) for 5 h and KF was lowered to 0.07%. The organic layer was concentrated to about 120 mL to obtain compound 1h hydrochloride in DCM solution (37.35 g, 94.7 A% purity).

### Step 2-6: Synthesis of compound 2 - 2,2'-(((1R,2R)-2-((carboxymethyl)(4-(2-oxo-2-(2,3,5,6-tetrafluorophenoxy)ethyl)benzyl)amino)cyclohexyl)azanediyl)diacetic acid

To a 1 L jacket reactor under N₂ protection, the solution of compound 1h hydrochloride obtained above was added. 4N HCl/dioxane (370 mL, 29.8 equiv, pre-dried by molecular sieves) was added. The mixture was warmed to 55-60 °C and stirred for 3 h at 55-60 °C. The reaction was monitored by HPLC (92.8 A% of compound 2, none of compound 2h remained). The reaction was cooled to 20-25 °C, filtered and the filter cake was washed with 50 mL DCM. The filter cake was dried under vacuum for 16 h at 45-50 °C. 26.35 g of compound 2 was obtained with 97.5 A% HPLC purity and 79.9 wt% assay in 70% corrected yield. Residual chloride is 5.2 wt%. E: HCl =1: 1. Residual dioxane is 11 wt%. E: dioxane = 1: 0.9. ¹H NMR (400MHz, DMSO-d₆) δ = 7.95 (tt, *J* =7.4*,* 10.9 Hz, 1H), 7.78-7.57 (m, 2H), 7.42 (br d, *J* =7.9 Hz, 1H), 7.33 - 7.15 (m, 1H), 4.50 (br d, *J* =12.7 Hz, 1H), 4.54 - 4.42 (m, 1H), 4.42 - 4.19 (m, 3H), 3.89 (br d, *J* =17.1 Hz, 2H), 3.66 - 3.50 (m, 9H), 3.50 - 2.96 (m, 4H), 2.23 (br d, *J* =10.5 Hz, 1H), 2.07 (br s, 1H), 1.72 (br d, *J* =18.7 Hz, 2H), 1.55 - 1.12 (m, 4H).

### Example 4: Salt screens and characterization of crystalline products

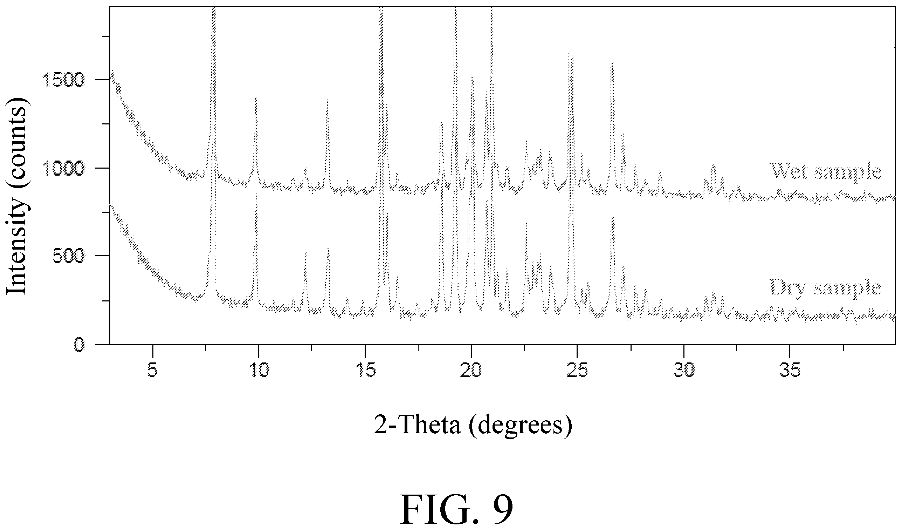

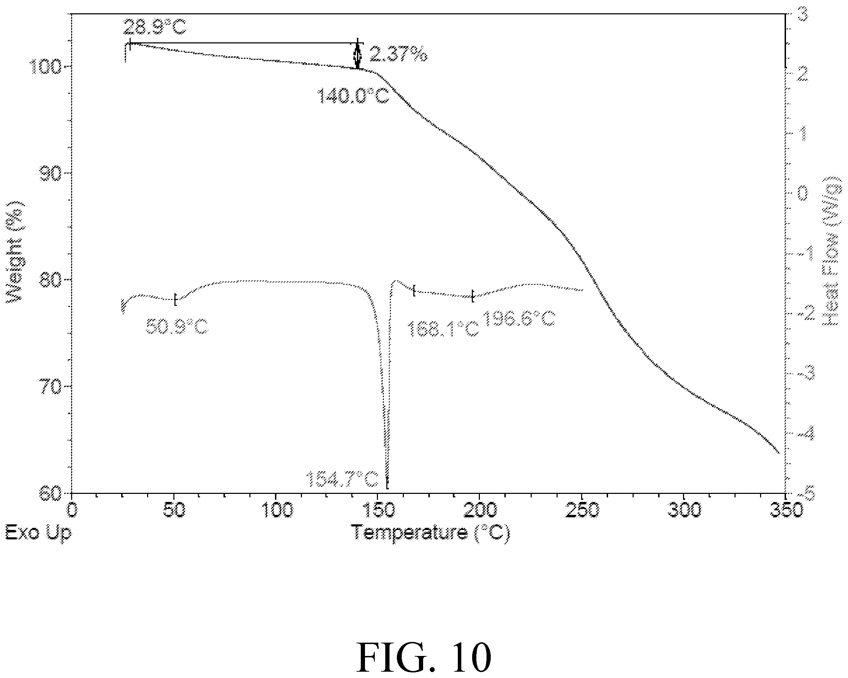

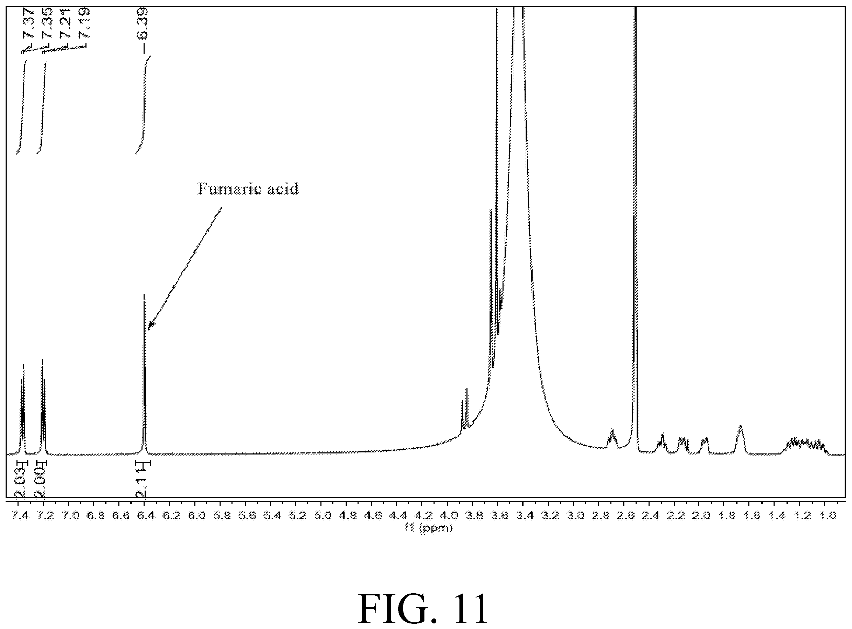

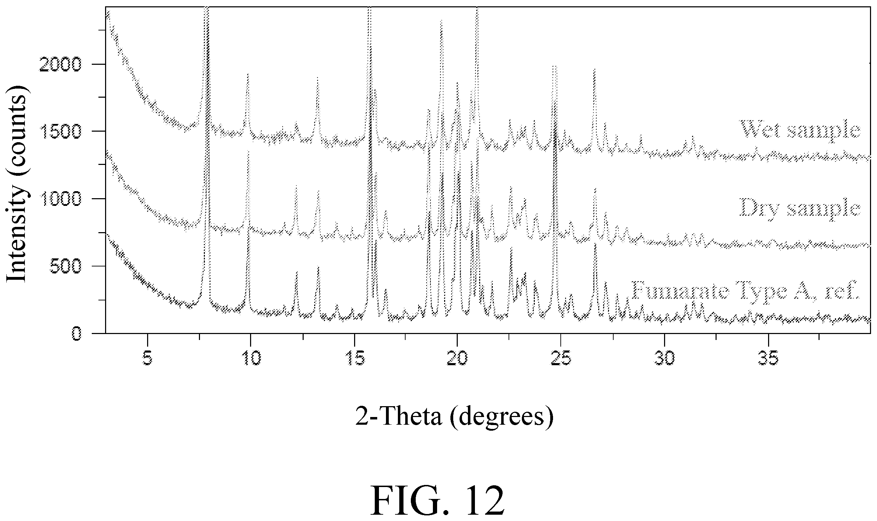

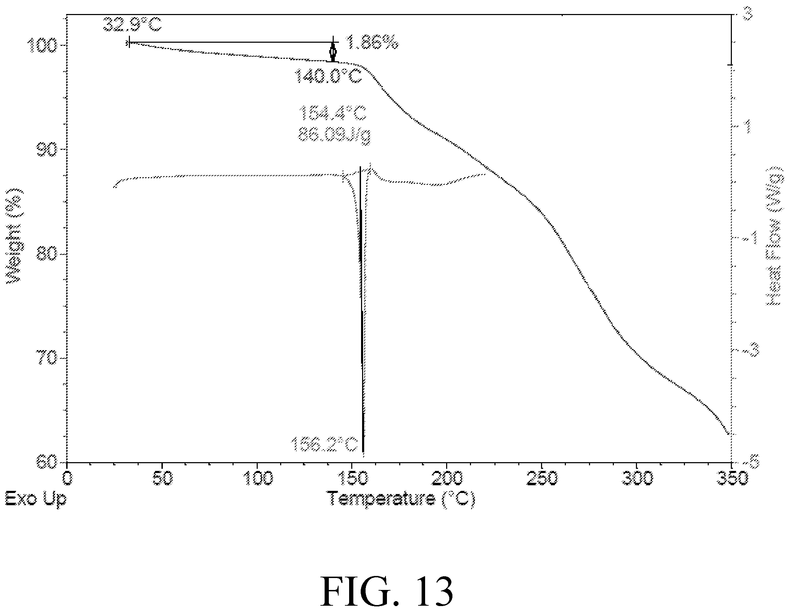

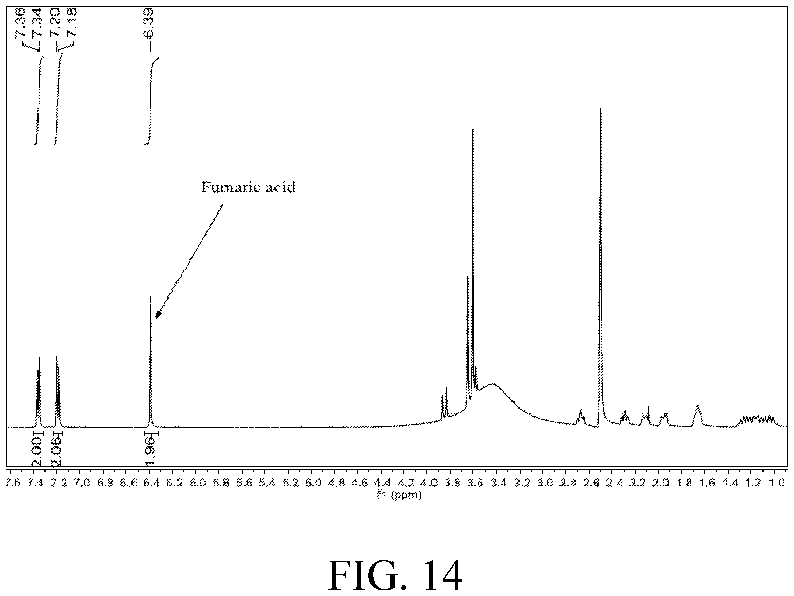

Starting from the oil-like compound 1c, a total of 51 salt screening experiments were performed using 17 acids in three solvent systems (*see* Table 1 below). 5 crystalline hits were obtained - fumarate type A, L-tartrate Type A, and orotate types A, B, and C. The L-tartrate hit was believed to degrade in air over time. The fumarate and orotate salts were characterized by X-Ray Power Diffraction (XRPD), thermo-gravimetric analysis (TGA), differential scanning calorimetry (DSC), and nuclear magnetic resonance (NMR) (*see* FIGs. 1-5 and 9-11). Three crystalline samples from 1,5-naphthalenedisulfononic acid, gallic acid, and gentisic acid systems were speculated to be degradation products. Based on the properties, Fumarate Type A and Orotate Type A were re-prepared at about a 200 mg scale by slurry and re-characterized by the same techniques as above (*see* FIGs. 6-8 and 12-15). Dry XRPD samples were dried under vacuum for at least 2 hours.

**Table 1. Results of salt screen.**

| Salt / Solvent System | Acetone | 2-MeTHF | CH₃CN/H₂O (19:1, v/v) |
|---|---|---|---|
| Blank | Gel* | Gel* | Gel* |
| HCl | Gel* | Gel* | Gel* |
| Citric Acid | Gel* | Gel* | Gel* |
| Maleic Acid | Gel* | Gel* | Gel* |
| Fumaric Acid | **Fumarate Type A** | Amorphous | Gel* |
| L-Maleic Acid | Gel* | Amorphous* * | Gel* |
| L-Tartaric Acid | Amorphous | Amorphous | **L-Tartrate Type A**** |
| Adipic Acid | Gel* | Gel* | Gel* |
| Succinic Acid | Gel* | Gel* | Gel* |
| Mucic acid | Amorphous | Amorphous | Gel* |
| p-Toluenesufonic acid | Gel* | Gel* | Gel* |
| 4-aminobenzoic acid | Gel* | Gel* | Gel* |
| Gallic acid | Degradation product** | Gel* | Gel* |
| Orotic Acid | **Orotate Type A** | **Orotate Type B** | **Orotate Type A** |
| 1,5-naphthalenedisulfonic acid | Degradation product* | Gel* | Gel* |
| Benzenesulfonic acid | Gel* | Gel* | Gel* |
| Cinnamic acid | Gel* | Gel* | Gel* |

| | | | |
|---|---|---|---|
| *: A clear solution was obtained from slurry at 5 °C, so slow evaporation at room temp was performed **: Obtained from slurry at 5 °C | | | |

## Claims

1. A method of preparing a compound of Formula (I): 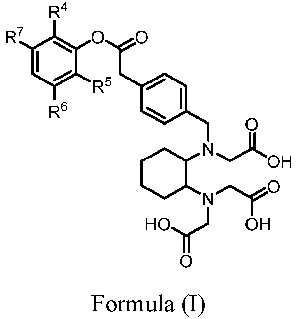
a diastereomer thereof, a salt thereof, a solvate thereof, or a combination of any of the foregoing,
wherein the method comprises:
(a) a step (1-1) comprising reacting a compound of Formula (Ia): 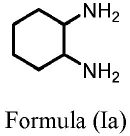
or a diastereomer thereof with a compound of Formula (Ib): 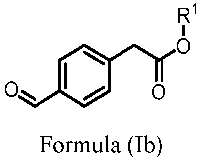
in a solvent and in the presence of a reducing agent to form a compound of Formula (Ic): 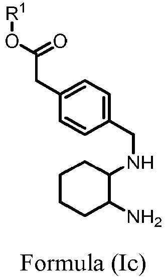
or a diastereomer thereof;
(b) a step (1-2) comprising reacting the compound of Formula (Ic), or a salt thereof, with a compound of Formula (Id): 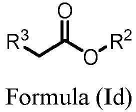
in the presence of a base and in a solvent,
to form a compound of Formula (Ie): 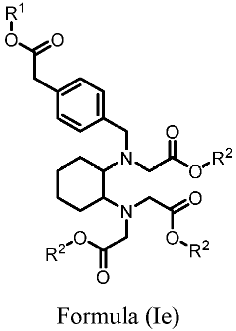
or a diastereomer thereof;
(c) a step (1-3) comprising reacting the compound of Formula (Ie) with a base in a solvent to form a compound of Formula (If): 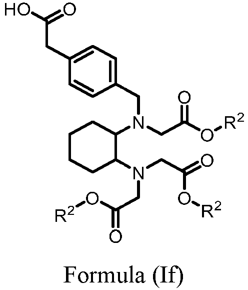 or a diastereomer thereof;
(d) a step (1-4) comprising reacting the compound of Formula (If) with a compound of Formula (1g): 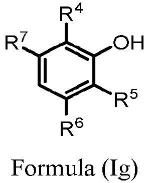
in a solvent and in the presence of a carboxyl activating reagent, to form a compound of Formula (Ih): 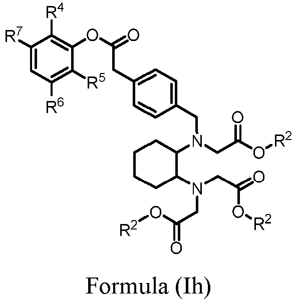
or a diastereomer thereof; and
(e) a step (1-5) comprising reacting the compound of Formula (Ih) with an acid to form a compound of Formula (I), a diastereomer thereof, a salt thereof, or a solvate thereof, or a combination of any of the foregoing,
wherein
R¹ and R² are each independently C₁-C₆ alkyl;
R³ is halo; and
R⁴, R⁵, R⁶ and R⁷ are each independently halo or hydrogen.

2. The method of claim 1, wherein the compound of Formula (I) is Compound 1:

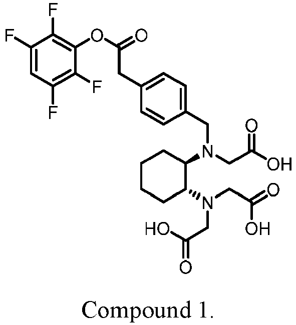

3. A method of preparing a compound of Formula (I): 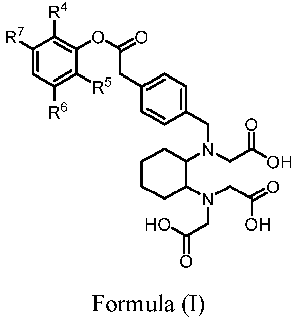
a diastereomer thereof, a salt thereof, or a solvate thereof, or a combination of any of the foregoing,
wherein the method comprises:
(a) a step (2-1) comprising reacting a compound of Formula (Ia): 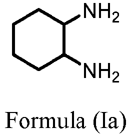
with a compound of Formula (Ib): 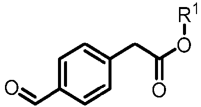 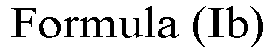
in a solvent and in the presence of a reducing agent to form a compound of Formula (Ic): 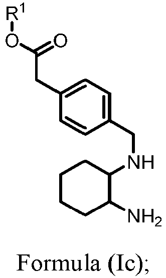
(b) a step (2-2) comprising reacting a compound of Formula (Ic) with an acid to produce a salt of Formula (Ic);
(c) a step (2-3) comprising reacting the salt of Formula (Ic) with a compound of Formula (Id): 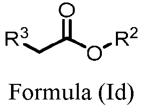
in the presence of a base and in a solvent,
to form a compound of Formula (Ie): 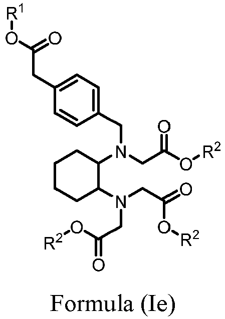
a diastereomer thereof, or a salt thereof;
(d) a step (2-4) comprising reacting a compound of Formula (Ie) with a base in a solvent to form a compound of Formula (2f): 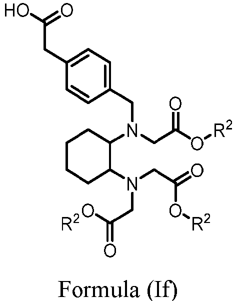 a diastereomer thereof, or a salt thereof; and
(e) a step (2-5) comprising reacting the compound of Formula (If) or salt thereof with a compound of Formula (Ig): 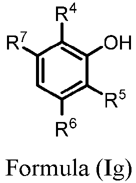
in a solvent and in the presence of a carboxyl activating reagent, to form a compound of Formula (Ih): 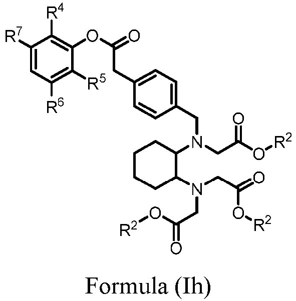
a diastereomer thereof, or a salt thereof; and
(f) a step (2-6) comprising reacting the compound of Formula (Ih) with an acid to form a compound of Formula (I), a diastereomer thereof, a salt thereof, a solvate thereof, or a combination of any of the foregoing,
wherein
R¹ and R² are each independently C₁-C₆ alkyl;
R³ is halo; and
R⁴, R⁵, R⁶, and R⁷ are each independently halo or hydrogen.

4. The method of claim 3, wherein the acid in the step (2-2) is orotic acid.

5. The method of any one of claims 3-4, wherein the step (2-2) provides a salt of Formula (Ic) having the following structure:

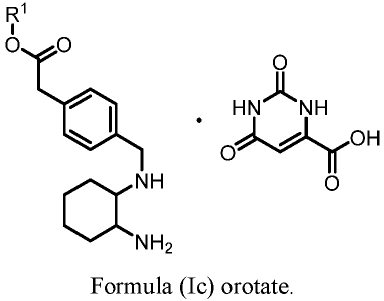

6. The method of claim 5, wherein the step (2-2) comprises crystallizing the salt of Formula (Ic), or a diastereomer thereof, as an orotate salt.

7. The method of any one of claims 3-6, wherein the step (2-6) provides a salt of Formula (I), or a solvate thereof, having the following structure:

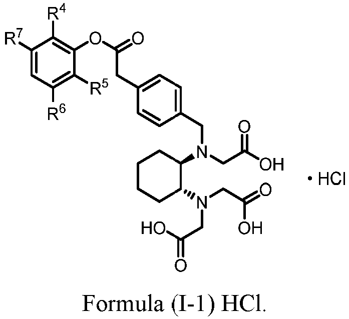

8. The method of claim 7, wherein the step (2-6) further comprises crystallizing the salt of Formula (I).

9. The method of any one of claims 3-8, wherein R¹ is methyl, or wherein R² is tert-butyl, or wherein R³ is Br, or wherein R⁴, R⁵, R⁶, and R⁷ are F.

10. The method of any one of claims 3-9, wherein the salt of the solvate of Formula (I) has the structure:

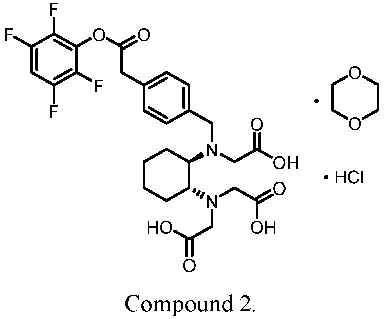

11. A compound of Formula (Ie): 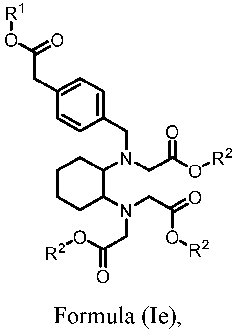
a diastereomer thereof, or a salt thereof,
wherein R¹ and R² are each independently C₁-C₆ alkyl.

12. A compound of Formula (Ic): 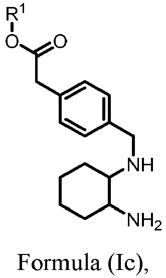
a diastereomer thereof, or a salt thereof,
wherein R¹ is C₁-C₆ alkyl.

13. A crystalline salt of the compound of claim 12 with the structure:

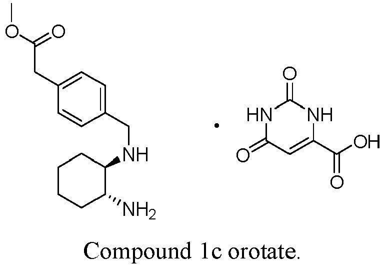

14. The crystalline salt of claim 13, wherein the salt has at least one of:
(a) an X-ray powder diffraction pattern with characteristic peaks at 7.5° 2-Theta, 13.9° 2-Theta, 16.6° 2-Theta, 23.4° 2-Theta, and 25.6° 2-Theta;
(b) a thermo-gravimetric analysis thermogram showing a mass loss of 3.3% between 20 °C and 150 °C;
(c) a differential scanning calorimetry thermogram showing an endothermic peak at 172.8 °C;
(d) an ¹H nuclear magnetic resonance spectrum with a characteristic peak at 5.68 ppm;
(e) an X-Ray powder diffraction pattern with characteristic peaks at 7.2° 2-Theta, 14.0° 2-Theta, 15.2° 2-Theta, 22.7° 2-Theta, and 25.0° 2-Theta;
(f) a thermo-gravimetric analysis thermogram showing a mass loss of 2.6% between 20 °C and 150 °C;
(g) a differential scanning calorimetry thermogram showing an exothermic peak at 161.7 °C and an endothermic peak at 182.9 °C;
(h) an ¹H nuclear magnetic resonance spectrum with a characteristic peak at 5.69 ppm;
(i) an X-Ray powder diffraction pattern with characteristic peaks at 7.3° 2-Theta, 7.8° 2-Theta, 9.7° 2-Theta, 13.9° 2-Theta, 16.7° 2-Theta, 21.4° 2-Theta, 23.4° 2-Theta;
(j) a thermo-gravimetric analysis thermogram showing a mass loss of 4.8% between 20 °C and 150 °C;
(k) a differential scanning calorimetry thermogram showing an exothermic peak at 178.1 °C and an endothermic peak at 185.6 °C;
(l) an ¹H nuclear magnetic resonance spectrum with a characteristic peak at 5.68 ppm.

15. A crystallized salt of the compound of claim 12 with the structure: 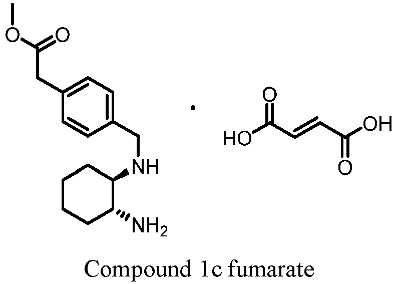 wherein the salt has at least one of:
(a) an X-Ray powder diffraction pattern with characteristic peaks at 7.9° 2-Theta, 15.8° 2-Theta, 18.6° 2-Theta, 19.3° 2-Theta, 20.1° 2-Theta, 21.0° 2-Theta, and 24.7° 2-Theta;
(b) a thermo-gravimetric analysis thermogram showing a mass loss of 2.4% between 20 °C and 140 °C;
(c) a differential scanning calorimetry thermogram showing an endothermic peak at 154.7 °C;
(d) an ¹H nuclear magnetic resonance spectrum with a characteristic peak at 6.39 ppm.

## Patentansprüche

1. Verfahren zur Herstellung einer Verbindung der Formel (I): 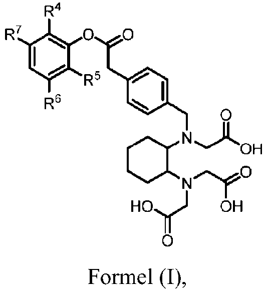 eines Diastereomers davon, eines Salzes davon, eines Solvats davon oder einer Kombination von beliebigen der Vorstehenden, wobei das Verfahren Folgendes umfasst:
(a) einen Schritt (1-1), umfassend das Umsetzen einer Verbindung der Formel (Ia): 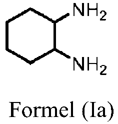
oder eines Diastereomers davon mit einer Verbindung der Formel (Ib): 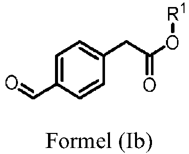
in einem Lösungsmittel und in Gegenwart eines Reduktionsmittels, um eine Verbindung der Formel (Ic) 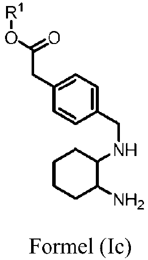
oder ein Diastereomer davon zu bilden;
(b) einen Schritt (1-2), umfassend das Umsetzen der Verbindung der Formel (Ic) oder eines Salzes davon mit einer Verbindung der Formel (Id): 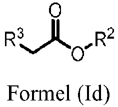
in Gegenwart einer Base und in einem Lösungsmittel,
um eine Verbindung der Formel (Ie) 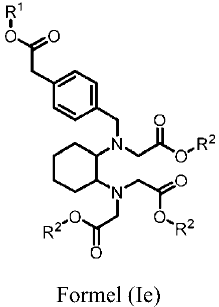
oder ein Diastereomer davon zu bilden;
(c) einen Schritt (1-3), umfassend das Umsetzen der Verbindung der Formel (Ie) mit einer Base in einem Lösungsmittel, um eine Verbindung der Formel (If) 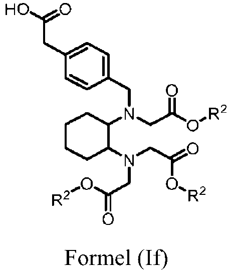 oder ein Diastereomer davon zu bilden;
(d) einen Schritt (1-4), umfassend das Umsetzen der Verbindung der Formel (If) mit einer Verbindung der Formel (Ig): 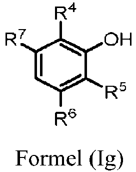
in einem Lösungsmittel und in Gegenwart eines Carboxylaktivierungsreagens, um eine Verbindung der Formel (Ih) 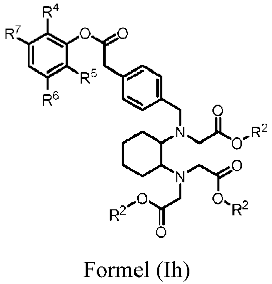
oder ein Diastereomer davon zu bilden; und
(e) einen Schritt (1-5), umfassend das Umsetzen der Verbindung der Formel (Ih) mit einer Säure, um eine Verbindung der Formel (I), ein Diastereomer davon, ein Salz davon oder ein Solvat davon oder eine Kombination von beliebigen der Vorstehenden zu bilden,
wobei
R¹ und R² jeweils unabhängig voneinander C₁-C₆-Alkyl sind;
R³ Halogen ist; und
R⁴, R⁵, R⁶ und R⁷ jeweils unabhängig voneinander Halogen oder Wasserstoff sind.

2. Verfahren nach Anspruch 1, wobei die Verbindung der Formel (I) Verbindung 1 ist:

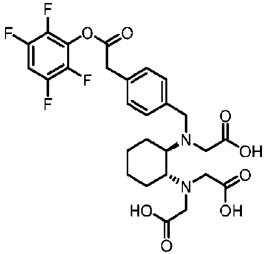

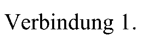

3. Verfahren zur Herstellung einer Verbindung der Formel (I): 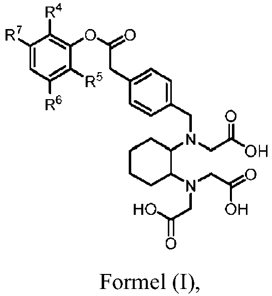
eines Diastereomers davon, eines Salzes davon oder eines Solvats davon oder einer Kombination von beliebigen der Vorstehenden,
wobei das Verfahren Folgendes umfasst:
(a) einen Schritt (2-1), umfassend das Umsetzen einer Verbindung der Formel (Ia): 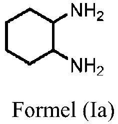
mit einer Verbindung der Formel (Ib): 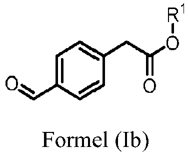
in einem Lösungsmittel und in Gegenwart eines Reduktionsmittels, um eine Verbindung der Formel (Ic) zu bilden: 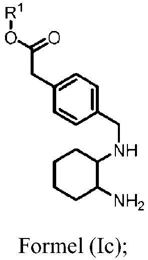
(b) einen Schritt (2-2), umfassend das Umsetzen einer Verbindung der Formel (Ic) mit einer Säure, um ein Salz der Formel (Ic) herzustellen;
(c) einen Schritt (2-3), umfassend das Umsetzen des Salzes der Formel (Ic) mit einer Verbindung der Formel (Id): 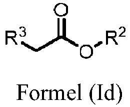
in Gegenwart einer Base und in einem Lösungsmittel,
um eine Verbindung der Formel (Ie) 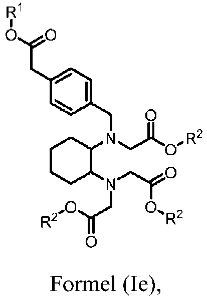
ein Diastereomer davon oder ein Salz davon zu bilden;
(d) einen Schritt (2-4), umfassend das Umsetzen einer Verbindung der Formel (Ie) mit einer Base in einem Lösungsmittel, um eine Verbindung der Formel (2f) 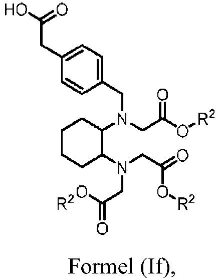 ein Diastereomer davon oder ein Salz davon zu bilden; und
(e) einen Schritt (2-5), umfassend das Umsetzen der Verbindung der Formel (If) oder des Salzes davon mit einer Verbindung der Formel (Ig): 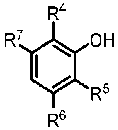 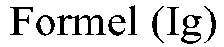
in einem Lösungsmittel und in Gegenwart eines Carboxylaktivierungsreagens, um eine Verbindung der Formel (Ih) 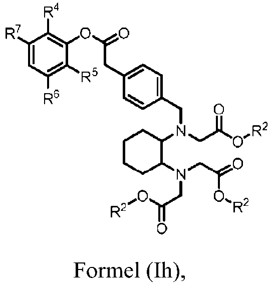
ein Diastereomer davon oder ein Salz davon zu bilden; und
(f) einen Schritt (2-6), umfassend das Umsetzen der Verbindung der Formel (Ih) mit einer Säure, um eine Verbindung der Formel (I), ein Diastereomer davon, ein Salz davon, ein Solvat davon oder eine Kombination von beliebigen der Vorstehenden zu bilden,
wobei
R¹ und R² jeweils unabhängig voneinander C₁-C₆-Alkyl sind;
R³ Halogen ist; und
R⁴, R⁵, R⁶ und R⁷ jeweils unabhängig voneinander Halogen oder Wasserstoff sind.

4. Verfahren nach Anspruch 3, wobei die Säure in Schritt (2-2) Orotsäure ist.

5. Verfahren nach einem der Ansprüche 3-4, wobei der Schritt (2-2) ein Salz der Formel (Ic) mit der folgenden Struktur bereitstellt:

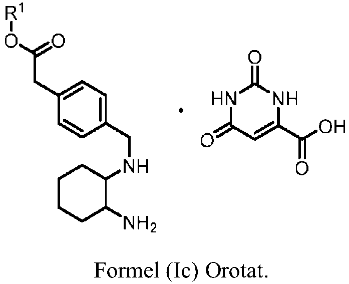

6. Verfahren nach Anspruch 5, wobei der Schritt (2-2) das Kristallisieren des Salzes der Formel (Ic) oder eines Diastereomers davon als ein Orotatsalz umfasst.

7. Verfahren nach einem der Ansprüche 3-6, wobei der Schritt (2-6) ein Salz der Formel (I) oder ein Solvat davon mit der folgenden Struktur bereitstellt:

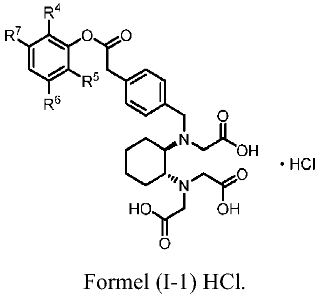

8. Verfahren nach Anspruch 7, wobei der Schritt (2-6) ferner das Kristallisieren des Salzes der Formel (I) umfasst.

9. Verfahren nach einem der Ansprüche 3-8, wobei R¹ Methyl ist oder wobei R² tert-Butyl ist oder wobei R³ Br ist oder wobei R⁴, R⁵, R⁶ und R⁷ F sind.

10. Verfahren nach einem der Ansprüche 3-9, wobei das Salz des Solvats der Formel (I) die folgende Struktur aufweist:

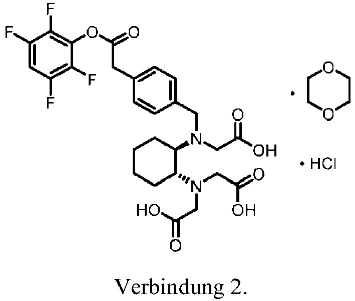

11. Verbindung der Formel (Ie): 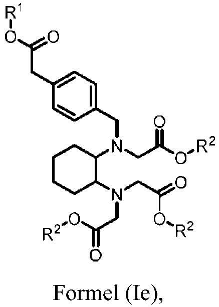
ein Diastereomer davon oder ein Salzes davon,
wobei R¹ und R² jeweils unabhängig voneinander C₁-C₆-Alkyl sind.

12. Verbindung der Formel (Ic): 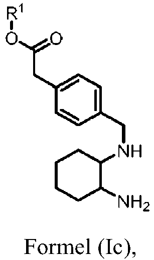
ein Diastereomer davon oder ein Salz davon,
wobei R¹ C₁-C₆-Alkyl ist.

13. Kristallines Salz der Verbindung nach Anspruch 12 mit der folgenden Struktur:

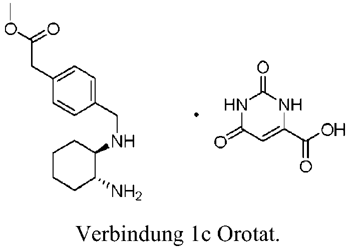

14. Kristallines Salz nach Anspruch 13, wobei das Salz mindestens eines der Folgenden aufweist:
(a) ein Röntgenpulverbeugungsmuster mit charakteristischen Peaks bei 7,5° 2-Theta, 13,9° 2-Theta, 16,6° 2-Theta, 23,4° 2-Theta und 25,6° 2-Theta;
(b) ein Thermogramm der thermogravimetrischen Analyse, das einen Massenverlust von 3,3 % zwischen 20 °C und 150 °C zeigt;
(c) ein Thermogramm der dynamischen Differenzkalorimetrie, das einen endothermen Peak bei 172,8 °C zeigt;
(d) ein ¹H-Kernmagnetresonanzspektrum mit einem charakteristischen Peak bei 5,68 ppm;
(e) ein Röntgenpulverbeugungsmuster mit charakteristischen Peaks bei 7,2° 2-Theta, 14,0° 2-Theta, 15,2° 2-Theta, 22,7° 2-Theta und 25,0° 2-Theta;
(f) ein Thermogramm der thermogravimetrischen Analyse, das einen Massenverlust von 2,6 % zwischen 20 °C und 150 °C zeigt;
(g) ein Thermogramm der dynamischen Differenzkalorimetrie, das einen exothermen Peak bei 161,7 °C und einen endothermen Peak bei 182,9 °C zeigt;
(h) ein ¹H-Kernmagnetresonanzspektrum mit einem charakteristischen Peak bei 5,69 ppm;
(i) ein Röntgenpulverbeugungsmuster mit charakteristischen Peaks bei 7,3° 2-Theta, 7,8° 2-Theta, 9,7° 2-Theta, 13,9° 2-Theta, 16,7° 2-Theta, 21,4° 2-Theta und 23,4° 2-Theta;
(j) ein Thermogramm der thermogravimetrischen Analyse, das einen Massenverlust von 4,8 % zwischen 20 °C und 150 °C zeigt;
(k) ein Thermogramm der dynamischen Differenzkalorimetrie, das einen exothermen Peak bei 178,1 °C und einen endothermen Peak bei 185,6 °C zeigt;
(l) ein ¹H-Kernmagnetresonanzspektrum mit einem charakteristischen Peak bei 5,68 ppm.

15. Kristallisiertes Salz der Verbindung nach Anspruch 12 mit der folgenden Struktur: 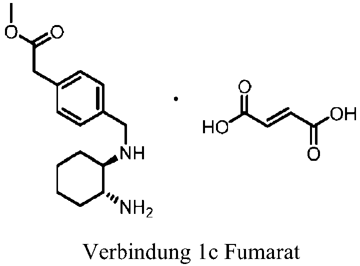 wobei das Salz mindestens eines der Folgenden aufweist:
(a) ein Röntgenpulverbeugungsmuster mit charakteristischen Peaks bei 7,9° 2-Theta, 15,8° 2-Theta, 18,6° 2-Theta, 19,3° 2-Theta, 20,1° 2-Theta, 21,0° 2-Theta und 24,7° 2-Theta;
(b) ein Thermogramm der thermogravimetrischen Analyse, das einen Massenverlust von 2,4 % zwischen 20 °C und 140 °C zeigt;
(c) ein Thermogramm der dynamischen Differenzkalorimetrie, das einen endothermen Peak bei 154,7 °C zeigt;
(d) ein ¹H-Kernmagnetresonanzspektrum mit einem charakteristischen Peak bei 6,39 ppm.

## Revendications

1. Procédé de préparation d'un composé de formule (I) : 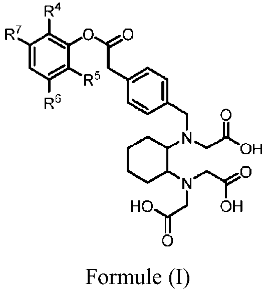 d'un diastéréoisomère de celui-ci, d'un sel de celui-ci, d'un solvate de celui-ci, ou d'une combinaison de n'importe lesquels des précédents, dans lequel le procédé comprend :
(a) une étape (1-1) comprenant la réaction d'un composé de formule (Ia) : 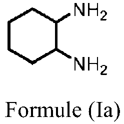
ou d'un diastéréoisomère de celui-ci avec un composé de formule (Ib) : 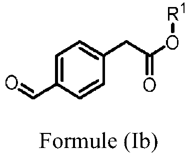
dans un solvant et en présence d'un agent réducteur pour former un composé de formule (Ic) : 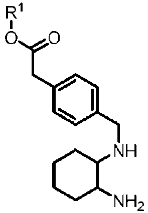 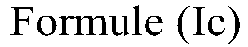
ou un diastéréoisomère de celui-ci ;
(b) une étape (1-2) comprenant la réaction du composé de formule (Ic), ou d'un sel de celui-ci, avec un composé de formule (Id) : 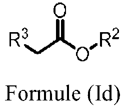
en présence d'une base et dans un solvant,
pour former un composé de formule (Ie) : 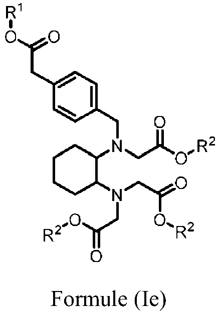
ou un diastéréoisomère de celui-ci ;
(c) une étape (1-3) comprenant la réaction du composé de formule (Ie) avec une base dans un solvant pour former un composé de formule (If) : 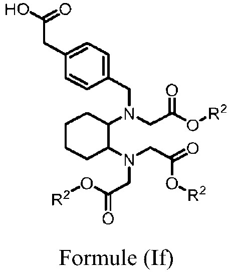 ou un diastéréoisomère de celui-ci ;
(d) une étape (1-4) comprenant la réaction du composé de formule (If) avec un composé de formule (Ig) : 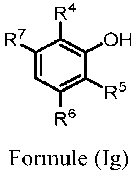
dans un solvant et en présence d'un réactif d'activation de carboxyle, pour former un composé de formule (Ih) : 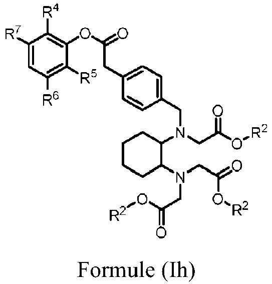 ou un diastéréoisomère de celui-ci ; et
(e) une étape (1-5) comprenant la réaction du composé de formule (Ih) avec un acide pour former un composé de formule (I), un diastéréoisomère de celui-ci, un sel de celui-ci ou un solvate de celui-ci, ou une combinaison de n'importe lesquels des précédents,
dans lequel
R¹ et R² représentent chacun indépendamment un alkyle en C₁-C₆ ;
R³ représente un halogène ; et
R⁴, R⁵, R⁶ et R⁷ représentent chacun indépendamment un halogène ou un hydrogène.

2. Composé selon la revendication 1, dans lequel le composé de formule (I) est le composé 1 :

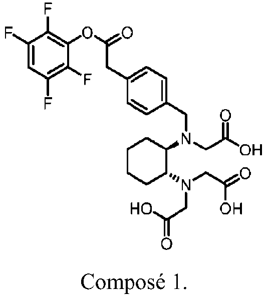

3. Procédé de préparation d'un composé de formule (I) : 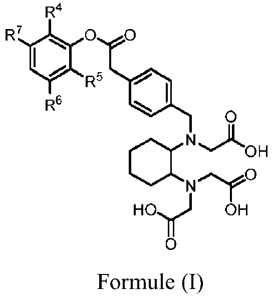
d'un diastéréoisomère de celui-ci, d'un sel de celui-ci ou d'un solvate de celui-ci, ou d'une combinaison de n'importe lesquels des précédents,
dans lequel le procédé comprend :
(a) une étape (2-1) comprenant la réaction d'un composé de formule (Ia) : 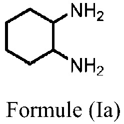
avec un composé de formule (Ib) : 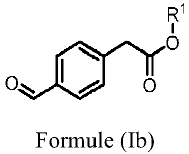
dans un solvant et en présence d'un agent réducteur pour former un composé de formule (Ic) : 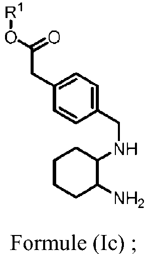
(b) une étape (2-2) comprenant la réaction d'un composé de formule (Ic) avec un acide pour produire un sel de formule (Ic) ;
(c) une étape (2-3) comprenant la réaction du sel de formule (Ic) avec un composé de formule (Id) : 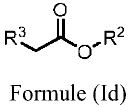
en présence d'une base et dans un solvant,
pour former un composé de formule (Ie) : 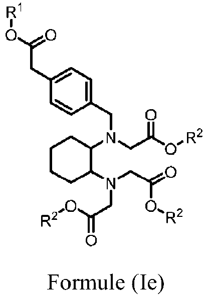
un diastéréoisomère de celui-ci, ou un sel de celui-ci ;
(d) une étape (2-4) comprenant la réaction d'un composé de formule (Ie) avec une base dans un solvant pour former un composé de formule (2f) : 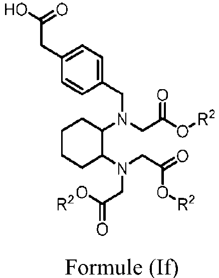 un diastéréoisomère de celui-ci ou un sel de celui-ci ; et
(e) une étape (2-5) comprenant la réaction du composé de formule (If) ou d'un sel de celui-ci avec un composé de formule (Ig) : 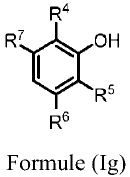
dans un solvant et en présence d'un réactif d'activation de carboxyle, pour former un composé de formule (Ih) : 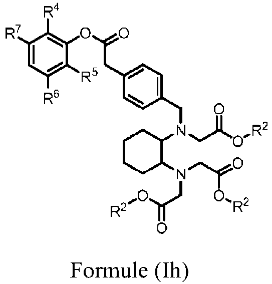
un diastéréoisomère de celui-ci ou un sel de celui-ci ; et
(f) une étape (2-6) comprenant la réaction du composé de formule (Ih) avec un acide pour former un composé de formule (I), un diastéréoisomère de celui-ci, un sel de celui-ci, un solvate de celui-ci, ou une combinaison de n'importe lesquels des précédents,
dans lequel
R¹ et R² représentent chacun indépendamment un alkyle en C₁-C₆ ;
R³ représente un halogène ; et
R⁴, R⁵, R⁶ et R⁷ représentent chacun indépendamment un halogène ou un hydrogène.

4. Procédé selon la revendication 3, dans lequel l'acide dans l'étape (2-2) est l'acide orotique.

5. Procédé selon l'une quelconque des revendications 3 à 4, dans lequel l'étape (2-2) fournit un sel de formule (Ic) ayant la structure suivante :

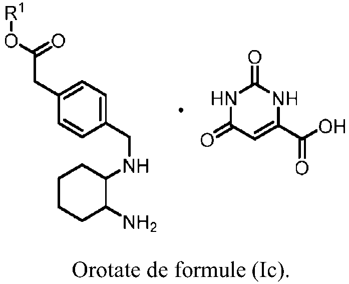

6. Procédé selon la revendication 5, dans lequel l'étape (2-2) comprend la cristallisation du sel de formule (Ic), ou d'un diastéréoisomère de celui-ci, en tant que sel d'orotate.

7. Procédé selon l'une quelconque des revendications 3 à 6, dans lequel l'étape (2-6) fournit un sel de formule (I), ou un solvate de celui-ci, ayant la structure suivante :

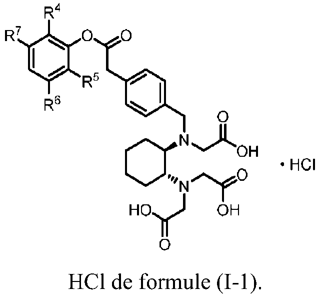

8. Procédé selon la revendication 7, dans lequel l'étape (2-6) comprend en outre la cristallisation du sel de formule (I).

9. Procédé selon l'une quelconque des revendications 3 à 8, dans lequel R¹ représente un méthyle, ou dans lequel R² représente un tert-butyle, ou dans lequel R³ représente Br, ou dans lequel R⁴, R⁵, R⁶ et R⁷ représentent F.

10. Procédé selon l'une quelconque des revendications 3 à 9, dans lequel le sel du solvate de formule (I) a la structure :

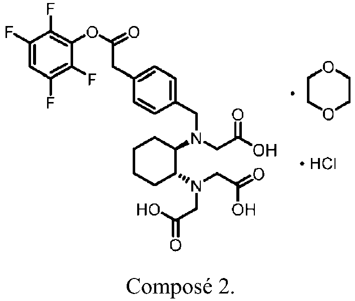

11. Composé de formule (Ie) : 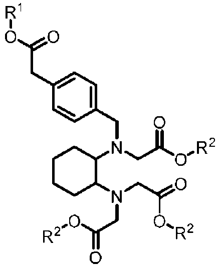 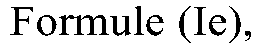
un diastéréoisomère de celui-ci ou un sel de celui-ci,
dans lequel R¹ et R² représentent chacun indépendamment un alkyle en C₁-C₆.

12. Composé de formule (Ic) : 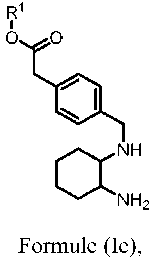
un diastéréoisomère de celui-ci ou un sel de celui-ci,
dans lequel R¹ représente un alkyle en C₁-C₆.

13. Sel cristallin du composé selon la revendication 12 ayant la structure :

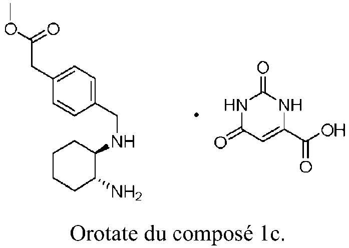

14. Sel cristallin selon la revendication 13, dans lequel le sel a au moins un parmi :
(a) un diagramme de diffraction des rayons X sur poudre avec des pics caractéristiques à 7,5° 2-thêta, 13,9° 2-thêta, 16,6° 2-thêta, 23,4° 2-thêta et 25,6° 2-thêta ;
(b) un thermogramme d'analyse thermogravimétrique montrant une perte de masse de 3,3 % entre 20 °C et 150 °C ;
(c) un thermogramme de calorimétrie différentielle à balayage montrant un pic endothermique à 172,8 °C ;
(d) un spectre de résonance magnétique nucléaire ¹H avec un pic caractéristique à 5,68 ppm ;
(e) un diagramme de diffraction des rayons X sur poudre avec des pics caractéristiques à 7,2° 2-thêta, 14,0° 2-thêta, 15,2° 2-thêta, 22,7° 2-thêta et 25,0° 2-thêta ;
(f) un thermogramme d'analyse thermogravimétrique montrant une perte de masse de 2,6 % entre 20 °C et 150 °C ;
(g) un thermogramme de calorimétrie différentielle à balayage montrant un pic exothermique à 161,7 °C et un pic endothermique à 182,9 °C ;
(h) un spectre de résonance magnétique nucléaire ¹H avec un pic caractéristique à 5,69 ppm ;
(i) un diagramme de diffraction des rayons X sur poudre avec des pics caractéristiques à 7,3° 2-thêta, 7,8° 2-thêta, 9,7° 2-thêta, 13,9° 2-thêta, 16,7° 2-thêta, 21,4° 2-thêta, 23,4° 2-thêta ;
(j) un thermogramme d'analyse thermogravimétrique montrant une perte de masse de 4,8 % entre 20 °C et 150 °C ;
(k) un thermogramme de calorimétrie différentielle à balayage montrant un pic exothermique à 178,1 °C et un pic endothermique à 185,6 °C ;
(l) un spectre de résonance magnétique nucléaire ¹H avec un pic caractéristique à 5,68 ppm.

15. Sel cristallisé du composé selon la revendication 12 ayant la structure : 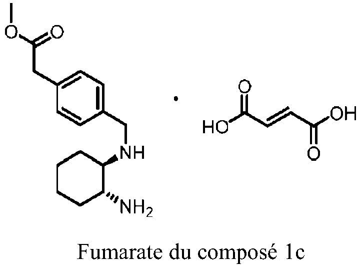 dans lequel le sel a au moins un parmi :
(a) un diagramme de diffraction des rayons X sur poudre avec des pics caractéristiques à 7,9° 2-thêta, 15,8° 2-thêta, 18,6° 2-thêta, 19,3° 2-thêta, 20,1° 2-thêta, 21,0° 2-thêta et 24,7° 2-thêta ;
(b) un thermogramme d'analyse thermogravimétrique montrant une perte de masse de 2,4 % entre 20 °C et 140 °C ;
(c) un thermogramme de calorimétrie différentielle à balayage montrant un pic endothermique à 154,7 °C ;
(d) un spectre de résonance magnétique nucléaire ¹H avec un pic caractéristique à 6,39 ppm.
